# EUROPÄISCHE PATENTSCHRIFT

(11) **EP 4 505 111 B1**
(45) Veröffentlichungstag und Bekanntmachung des Hinweises auf die Patenterteilung: **13.08.2025**
(21) Anmeldenummer: 22722415.1
(22) Anmeldetag: 07.04.2022
(51) Int. Cl.: F21V 29/58, F21V 31/00, F21V 29/503, F21V 29/508, F21V 23/00, C02F 1/32, F21Y 105/10, F21Y 115/10

(54) **FLÄCHENSTRAHLER, VORRICHTUNG MIT DEM FLÄCHENSTRAHLER UND VERWENDUNG DES FLÄCHENSTRAHLERS**
SURFACE RADIATOR, DEVICE COMPRISING THE SURFACE RADIATOR AND USE OF THE SURFACE RADIATOR
RADIATEUR DE SURFACE, DISPOSITIF COMPRENANT LE RADIATEUR DE SURFACE ET UTILISATION DU RADIATEUR DE SURFACE

(43) Veröffentlichungstag der Anmeldung: 12.02.2025
(73) Patentinhaber: Peschl Ultraviolet GmbH, 55130 Mainz (DE)
(72) Erfinder: PESCHL, Alexander, 55130 Mainz (DE)
(74) Vertreter: mepat Patentanwälte
(86) Internationale Anmeldenummer: PCT/EP2022/059279
(87) Internationale Veröffentlichungsnummer: WO 2023/193915

(56) Entgegenhaltungen:
- CN-A- 108 180 403
- DE-U1- 202014 103 329
- KR-A- 20160 100 712
- US-A1- 2017 030 567
- US-A1- 2020 124 266

## Beschreibung

Die Erfindung betrifft einen Flächenstrahler mit einem lichtemittierenden Halbleiterbauelement sowie eine mit einem solchen Flächenstrahler ausgestattete Vorrichtung zur Beleuchtung, zur Durchführung einer photochemischen Reaktion oder zur Desinfektion, und eine Verwendung eines solchen Flächenstrahlers auch zur Durchführung einer photochemischen Reaktion oder zur Desinfektion.

Aus dem Stand der Technik ist bekannt, Flächenstrahler mit gerichteter Lichtabstrahlung zu verwenden, um eine gleichmäßige Ausleuchtung einer Fläche zu erreichen. Ein Flächenstrahler hat daher eine einseitige Abstrahlcharakteristik, wobei der Abstrahlwinkel variieren kann. Wegen des deutlich geringeren Energieverbrauchs werden zunehmend Flächenstrahler mit Licht emittierenden Halbleiterbauelementen wie Licht emittierende Dioden (LED) auch zur großflächigen Abstrahlung von Licht eingesetzt. LEDs haben eine hohe Lebensdauer und hohe Schaltfestigkeit auch bei spontan vollem Lichtstrom. LEDs sind zwar keine Wärmestrahler, allerdings verkürzen hohe Temperaturen, die im Betrieb in Abhängigkeit der Anordnung und der Leistung der LEDs durchaus auftreten, die Lebensdauer der LEDs deutlich. Um diesen nachteiligen Einfluss zu vermeiden, werden LEDs häufig nicht bei Nennleistung, sondern darunter - verbunden mit geringerer Leuchtleistung - betrieben. Um dennoch eine gewünschte Lichtmenge zu erreichen, wird dann die Anzahl der eingesetzten LEDs erhöht.

Zur Bildung von Flächenstrahlern werden die LEDs auf einer im Wesentlichen ebenen, meist rechteckigen Trägerfläche mittels eines Gehäuserahmens hinter einem Emissionsfenster angeordnet und können zur Einstellung der Abstrahlcharakteristik von einem Reflektorrahmen umgeben sein. Bei Flächenstrahlern mit einer sehr großen Anzahl an LEDs können diese gruppenweise auf jeweils einer Platine befestigt werden, auf der zur elektrischen Kontaktierung der LEDs Leiterbahnen und ggf. zur Steuerung auch Vorschaltgeräte (LED-Treiber) vorgesehen sind. Die Leiterbahnen verlaufen üblicherweise zu einem Rand der Platine, um dort den elektrischen Anschluss zu ermöglichen. Nachteilig kann die im Betrieb entstehende Wärme von der Platine nur unzureichend abgeführt werden.

EP 2 701 473 A2 schlägt daher vor, für einen LED-Flächenstrahler eine Platine zu verwenden, die flächig ausgebildete Leiterbahn-Segmente bereitstellt, die eine vergleichsweise große Grenzfläche mit einer Umgebung der Platine ausbilden. Mit den flächig ausgebildeten Leiterbahn-Segmenten soll die von den LEDs erzeugte Abwärme von der Platine in die Umgebung abgeführt werden.

Zur deutlichen Verbesserung der Wärmeabfuhr werden Metallgehäuse, meistens aus Aluminium, eingesetzt. So ist z. B. aus DE 20 2011 050 253 U1 eine Beleuchtungsvorrichtung mit einer Mehrzahl von LED bekannt, die auf einer als Leiterplatte ausgebildeten Trägereinheit angeordnet ist. Die Beleuchtungsvorrichtung weist ferner einen H-förmigen Aluminium-Profilkörper mit zwei Abschnitten auf, wobei die Trägereinheit mit den LEDs auf einem ersten Abschnitt des Profilkörpers angeordnet ist. Eine lichtdurchlässige Polymerhülle umschließt die Trägereinheit mit den LEDs und den ersten Abschnitt. Der zweite Abschnitt des Profilkörpers, der aus der Polymerumhüllung herausragt, dient der Wärmeableitung an einen Gehäusekörper.

Im industriellen Einsatz mit Hochleistungsdioden, die für photochemische Reaktionen mit einer hohe Lichtausbeute bzw. Strahlungsintensität mit hohen Strömen betrieben werden, ist eine noch effektivere Wärmeableitung erforderlich, um die Lebensdauer der LEDs zu erhalten.

Die WO2020/148289 A1 beschreibt eine flächige Lichtquelle mit LEDs zum Härten von Druckfarben oder Lacken, wobei zumindest ein lichtemittierendes Halbleiterbauelement (als LED-Modul mit einer zugehörigen Platine) auf einer Trägerplatte angeordnet ist. Die Lichtquelle weist ferner ein als Gehäuse ausgebildetes Verteilerelement auf, an dem ein Emissionsfenster angeordnet ist, das das zumindest eine LED-Modul überlagert. Ferner weist das Gehäuse-Verteilerelement einen Kühlkanal auf, der über ein Verbindungselement mit Anschlüssen für den Zulauf und Rücklauf eines Kühlfluids verbunden ist. Auf dem Gehäuse-Verteilerelement können mehrere Trägerplatten mit LED-Modulen angeordnet werden, wobei eine von dem LED-Modul abgewandte Oberfläche jeder Trägerplatte als Kühloberfläche ausgebildet ist, die abgedichtet an dem Verteilerelement angeordnet ist. Die Kühloberfläche begrenzt so teilweise einen Fluidweg, der mit dem Kühlkanal im Verteilerelement über Kanalabzweige verbunden ist.

DE 20 2014 103329 U1 betrifft einen Scheinwerfer mit einer ebenfalls flüssigkeitsgekühlten LED-Lichtquelle. Zur Verbesserung der Kühlung kann die LED-Lichtquelle an einem Kühler mit separater Flüssigkeitskühlung angeordnet oder an einem Kühlelement befestigt sein, das mit der LED-Lichtquelle innerhalb des Gehäuses angeordnet und ebenfalls von dem Kühlmittel umströmt wird.

US 2017/030567 A1 offenbart ebenfalls eine flüssigkeitsgekühlte LED-Leuchte, und aus CN 108 180 403 A ist eine flüssigkeitsgekühlte Laserlicht-emittierende Vorrichtung bekannt.

KR 2016 0100712 A offenbart einen LED-Flächenstrahler mit einem Gehäusekörper, in dem zwei Kühlkanäle ausgebildet sind, die durch eine Metallplatte mit darauf befestigten LEDs abgedeckt sind. Beidseitig benachbart zu den LEDs sind in der Metallplatte Fluidöffnungen ausgebildet, die jeweils mit einem der beiden Kühlkanäle kommunizieren. Ein von der Platine mit den LEDs beabstandetes Emissionsfenster ist fluiddicht mit dem Gehäusekörper verbunden, sodass sich ein Fluidweg für ein elektrisch isolierendes und transparentes Kühlmittel zwischen den Kühlkanälen durch die Fluidöffnungen entlang den LEDs erstreckt.

Ausgehend von diesem Stand der Technik ist es Aufgabe der vorliegenden Erfindung, einen Flächenstrahler bereitzustellen, der hinsichtlich der Wärmeabfuhr und der thermischen Abkopplung der lichtemittierenden Halbleiterelemente verbessert ist.

Diese Aufgabe wird durch einen Flächenstrahler mit den Merkmalen des Anspruchs 1 gelöst.

Die weitere Aufgabe, eine hinsichtlich der Wärmeabfuhr und der thermischen Abkopplung der lichtemittierenden Halbleiterelemente eines Flächenstrahlers verbesserte Vorrichtung zur Beleuchtung, Durchführung einer photochemischen Reaktion oder Desinfektion bereitzustellen, wird durch eine Vorrichtung mit den Merkmalen des unabhängigen Anspruchs 10 gelöst.

Eine Verwendung eines Flächenstrahlers, der hinsichtlich der Wärmeabfuhr und der thermischen Abkopplung der lichtemittierenden Halbleiterelemente verbessert ist, wird durch die Verwendung der Vorrichtung mit den Merkmalen des unabhängigen Anspruchs 12 offenbart.

Weiterbildungen sind in den jeweiligen Unteransprüchen ausgeführt.

Gemäß einer ersten Ausführungsform weist ein erfindungsgemäßer Flächenstrahler zumindest ein lichtemittierendes Halbleiterbauelement und einen Gehäusekörper mit zumindest einem Kühlkanal für ein Kühlmittel auf. Dabei umfasst ein Flächenstrahler üblicherweise eine Mehrzahl lichtemittierender Halbleiterelemente (LEDs), die für eine einseitige gerichtete Lichtabstrahlung nebeneinander in gleichmäßigen Abständen auf einer im Wesentlichen ebenen Fläche angeordnet sind. Der Kühlkanal bildet zumindest einen Teil eines Fluidwegs, der sich von einer Zulauföffnung für das Kühlmittel zu einer Rücklauföffnung für das Kühlmittel erstreckt. Die Zulauföffnung und die Rücklauföffnung sind an dem Gehäusekörper ausgebildet und vorzugsweise zum Anschluss an einen Kühlmittelkreislauf vorgesehen. An dem Gehäusekörper ist ein Emissionsfenster angeordnet, das eine Vorderseite des Flächenstrahlers definiert, an dem die einseitige gerichtete Lichtabstrahlung erfolgt. Als "Emissionsfenster" wird hierbei eine Platte oder Scheibe aus einem Material verstanden, das für eine von dem zumindest einen lichtemittierenden Halbleiterbauelement emittierte Einsatzstrahlung transparent ist, die - je nach vorgesehenem Einsatz des Flächenstrahlers zur Beleuchtung, zur Durchführung einer photochemischen Reaktion oder zur Desinfektion - durch zumindest eine vorbestimmte Wellenlänge oder zumindest einen vorbestimmten Wellenlängenbereich elektromagnetischer Strahlung definiert ist. "Transparent" bedeutet hierbei, dass das Emissionsfenster für Einsatzstrahlung einen Transmissionsgrad von zumindest 75 % aufweist. Die Dimensionierung des Emissionsfensters und dessen Anordnung an dem Gehäusekörper korrespondiert mit der Anordnung der einen LED oder den mehreren LEDs, sodass das Emissionsfenster das zumindest eine lichtemittierende Halbleiterbauelement flächig überlagert.

Dabei stellt der Gehäusekörper selbst, ohne dass eine zusätzliche Trägerstruktur wie im Stand der Technik erforderlich ist, eine von dem Emissionsfenster beabstandete Befestigungsfläche für das zumindest eine lichtemittierende Halbleiterbauelement bereit, wobei die Befestigungsfläche zur Anordnung der LED(s) mit dem Emissionsfenster korrespondiert. Dabei ist die Anordnung des Emissionsfensters an dem Gehäusekörper fluiddicht ausgebildet, sodass durch den Gehäusekörper, das Halbleiterbauelement und das Emissionsfenster ein abgedichteter Emissionsraum begrenzt wird. Der zur Kühlung der LED vorgesehene Fluidweg wird dann definiert durch
i) zumindest einen ersten Kühlkanal, der sich von der Zulauföffnung durch den Gehäusekörper zu einer oder gegebenenfalls mehreren Mündungsöffnung(en) erstreckt, die auf einer ersten Seite neben der Befestigungsfläche ausgebildet ist und in den Emissionsraum mündet, und
ii) den Emissionsraum von der Mündungsöffnung bis zu einer oder mehreren Ablauföffnung(en), die auf einer von der ersten Seite abgewandten zweiten Seite neben der Befestigungsfläche im Gehäusekörper ausgebildet ist bzw. sind, sodass die Befestigungsfläche zwischen der Mündungsöffnung und der Ablauföffnung liegt und der Emissionsraum komplett mit dem Kühlmittel gefüllt wird, und
iii) zumindest einen zweiten Kühlkanal, der sich von der Ablauföffnung durch den Gehäusekörper zu der Rücklauföffnung erstreckt.

Da somit erfindungsgemäß das Kühlmittel den Emissionsraum flutet und mit den LEDs in direkten Kontakt kommt, um die im Betrieb von den LEDs erzeugte Wärme effektiver abzuleiten, ist erfindungsgemäß das Kühlmittel eine elektrisch isolierende Flüssigkeit, die für die von dem lichtemittierenden Halbleiterbauelement emittierte Einsatzstrahlung transparent ist und aufgrund der spezifischen Wärmekapazität, Dichte und des Wärmeleitkoeffizienten für einen besseren Wärmeübergang als mit einem gasförmigen Kühlmedium oder Luft sorgt. Unter Transparenz wird hierin ein Transmissionsgrad von zumindest 75 % für die Einsatzstrahlung in Bezug auf die Weglänge verstanden, die durch den Abstand zwischen LED und Emissionsfenster definiert wird. Vorteilhaft kann durch die Flutung des Emissionsraums mit dem flüssigen Kühlmittel, das aus dem ersten Kühlkanal durch die Mündungsöffnung zugeführt und durch die Ablauföffnung in den zweiten Kühlkanal abgeführt wird, nicht nur Wärme von den LEDs aufgenommen werden, sondern es kann über das Emissionsfenster gegebenenfalls auch Wärme aus der Umgebung aufgenommen werden, die nach außerhalb des Flächenstrahlers abgegeben werden kann, sodass ein Wärmestau zwischen LEDs und Emissionsfenster vermieden, die Temperatur der LED konstant gehalten und eine thermische Abkopplung des Flächenstrahlers von der Umgebung erreicht werden. Ein weiterer Vorteil der Flutung des Emissionsraums mit dem flüssigen Kühlmittel besteht in der Vermeidung von Kondenswasserbildung im Fall eines großen Temperaturgefälles zur Umgebung bzw. der bestrahlten Oberfläche.

Erfindungsgemäß ist an der Vorderseite in dem Gehäusekörper benachbart zu der Befestigungsfläche eine zu dem Emissionsraum geöffnete Anschlusskammer ausgebildet. Dabei ist an dem Gehäusekörper eine Anschlussöffnung ausgebildet, die mit der Anschlusskammer verbunden ist, wobei sich zumindest eine Anschlussleitung zum Anschluss des zumindest einen lichtemittierenden Halbleiterbauelements zumindest in die Anschlusskammer erstreckt. Ein Vorschaltgerät ist entweder auf einer Platine vorgesehen, mittels derer eine Mehrzahl der lichtemittierenden Halbleiterbauelemente an der Befestigungsfläche befestigt ist. An einer der Anschlusskammer nahen Seite der Platine ist auf der Platine jeweils ein Paar Anschlusskontakte für eine Reihe lichtemittierender Halbleiterbauelemente ausgebildet, wobei jeder Anschlusskontakt mit einer jeweiligen Anschlussleitung verbunden ist, wobei das Vorschaltgerät zwischen den Anschlusskontakten und den lichtemittierenden Halbleiterbauelementen (LEDs) vorgesehen ist. Oder das Vorschaltgerät für das zumindest eine lichtemittierende Halbleiterbauelement ist verbunden mit der zumindest einen Anschlussleitung in der Anschlusskammer angeordnet.

D. h., dass an der Vorderseite in dem Gehäusekörper benachbart zu der Befestigungsfläche eine zu dem Emissionsraum geöffnete Anschlusskammer zur Aufnahme von elektrischen Anschlusselementen für die LED ausgebildet sein kann, die wie der Emissionsraum mit dem Kühlmittel gefüllt ist. Ferner ist an dem Gehäusekörper eine Anschlussöffnung ausgebildet, wobei die Anordnung der Anschlussöffnung an einer der Seitenflächen oder der Rückseite des Gehäusekörpers wie die Anordnung der Zulauf- und Rücklauföffnung in Abhängigkeit eines Einbaukontextes angepasst ausgewählt werden kann. Als elektrische Anschlusselemente zur Verbindung mit einer Stromversorgung außerhalb des Gehäusekörpers erstreckt sich zumindest eine Anschlussleitung zum Anschluss des zumindest einen lichtemittierenden Halbleiterbauelements zumindest in die Anschlusskammer, und ggf. in oder durch die Anschlussöffnung.

Ferner weist ein erfindungsgemäßer Flächenstrahler mehrere lichtemittierende Halbleiterbauelemente auf, insbesondere mehrere LEDs, die auf einer Platine angeordnet sind, ggfs. auf mehreren, mit der die lichtemittierenden Halbleiterbauelemente an der Befestigungsfläche des Gehäusekörpers befestigt sind. Dabei ist auf der Platine jeweils ein Paar Anschlusskontakte für eine Reihe lichtemittierender Halbleiterbauelemente an einer der Anschlusskammer nahen Seite der Platine ausgebildet, wobei jeder Anschlusskontakt mit einer jeweiligen Anschlussleitung elektrisch leitend verbunden ist. Eine "Reihe" LEDs bezeichnet hierbei eine vorbestimmte Anzahl LEDs auf einer Platine, die vorzugsweise über die Länge einer Platine auf einer Geraden angeordnet sein können, aber ggf. auch in einem anderen Muster, z. B. im Zickzack angeordnet sein können. Ein Flächenstrahler kann eine Platine mit einer Reihe LEDs, vorzugsweise mit mehreren parallelen Reihen LEDs aufweisen. Durch die Anordnung der Anschlusskontakte auf der Seite der Anschlusskammer kann die Länge der Anschlussleitungen minimiert und eine Leitungsführung durch den Emissionsraum vermieden werden, wodurch nicht nur eine Verschattung der LEDs verhindert wird, sondern auch Leitungsmantelmaterialien z. B. vor UV-Strahlung geschützt werden können. Die elektrisch leitende Verbindung einer Anschlussleitung an einem Anschlusskontakt kann zur Vermeidung von Isolationswiderständen durch das Kühlmittel z. B. verlötet sein.

Ein für den Betrieb der LEDs erforderliches Vorschaltgerät bzw. LED-Treiber kann auf der Platine zwischen den Anschlusskontakten und den LEDs vorgesehen sein. Um jedoch einen Wärmeeintrag eines Vorschaltgeräts auf die LEDs zu minimieren, ist bevorzugt vorgesehen, dass das Vorschaltgerät für das lichtemittierende Halbleiterbauelement in der Anschlusskammer angeordnet und mit der einen oder mehreren Anschlussleitung verbunden ist. Die von dem Vorschaltgerät erzeugte Wärme wird dabei nicht über die Platine zu den LEDs geleitet, sondern über das Kühlmittel an den Gehäusekörper übertragen, der die Anschlusskammer umgibt und/oder mit dem Kühlmittel aus dem Emissionsraum abgeführt.

Bevorzugt kann eine Kreislaufführung des Kühlmittels vorgesehen werden, in dem das im Flächenstrahler erwärmte Kühlmittel im Kreislauf geführt wird, um außerhalb des Emissionsraums die aufgenommene Wärme wieder abzugeben. Weiter vorteilhaft sorgt die Flutung des Emissionsraums mit dem flüssigen Kühlmittel für eine Vermeidung von Nahfeldreflexion zwischen LED und Emissionsfenster und verhindert die Präsenz von flüchtigen organischen Kohlenwasserstoffverbindungen (VOCs) im Emissionsraum, die die LEDs schädigen könnten. Vorteilhaft ist dabei im Gegensatz zur Verwendung von einem Inertgas wie Stickstoff, dass eine beschleunigte Alterung der Primäroptiken der LEDs vermieden wird. Denn gerade in Chemieanlagen sind VOCs ("volatile organic compounds") vorhanden, die in die üblicherweise als Silikonlinse ausgeführten Primäroptiken eindringen, diese eintrüben und somit die Lichtausbeute senken. Da die Primäroptiken erfindungsgemäß keiner gashaltigen Atmosphäre mehr ausgesetzt sind, sondern durch das Kühlmittel abgeschirmt werden, wird der Alterungsprozess deutlich verlangsamt. Um die Einschränkungen in der Lichtausbeute infolge des Alterungsprozesses der Primäroptiken gänzlich zu vermeiden, besteht bei einem erfindungsgemäßen Flächenstrahler vorteilhaft die Möglichkeit, dass bei den LEDs auf schützende Primäroptiken wie Silikonlinsen verzichtet werden kann, da durch das flüssige Kühlmittel der Halbleiterchip der LEDs bereits ausreichend vor Umwelteinflüssen geschützt ist.

Ein erfindungsgemäßer Flächenstrahler ist dadurch nicht nur hinsichtlich der Kühlung der LEDs und der thermischen Abkopplung von der Umgebung verbessert, sondern sichert auch die LEDs vor schädlichen Substanzen. Darüber hinaus stellt ein erfindungsgemäßer Flächenstrahler besonders vorteilhaft eine erhöhte Gesamtlicht- bzw. Strahlungsleistung in Bezug auf die Lampen gemäß Stand der Technik bereit, da aufgrund des flüssigen Kühlmittels die Photonen-Auskopplungseffizienz an der Phasengrenze der Diodenoberfläche zum Kühlmittel im Emissionsraum vergrößert und die Reflexion an der Phasengrenze zwischen dem im Emissionsraum vorliegenden Kühlmittel und dem Emissionsfenster verringert wird.

Unter "Einsatzstrahlung" wird vorliegend elektromagnetische Strahlung bestimmter Wellenlängen oder Wellenlängenbereichen verstanden, die für einen vorbestimmten Einsatz des Flächenstrahlers, z. B. zur Beleuchtung, Durchführung einer photochemischen Reaktion oder zur Desinfektion geeignet sind.

Ein lichtemittierendes Halbleiterbauelement kann eine lichtemittierende Diode (LED) oder vorzugsweise ein LED-Modul sein, das aus zumindest einer LED und einer Platine besteht, die Leiterbahnen zum elektrischen Anschluss der zumindest einen LED aufweist. Eine Platine entspricht dabei nicht einer Trägerstruktur aus dem Stand der Technik: Dort werden ebenfalls LED-Module eingesetzt, die aus zumindest einer LED und einer Platine bestehen, wobei die Module mittels der Platine auf den Trägerstrukturen, wie die H-förmigen Trägereinheiten oder die Trägerplatten mit Kühloberflächen, befestigt werden, und die Trägerstrukturen an einem Gehäusekörper angeordnet werden.

Unter LEDs werden vorliegend sämtliche lichtemittierende Dioden, auch organische Leuchtdioden (OLEDs), verstanden, die elektromagnetische Strahlung in den infraroten, sichtbaren oder ultravioletten Wellenlängenbereichen emittieren. Ferner können LEDs ein Emissionsspektrum mit kombinierten Bestandteilen in den infraroten, sichtbaren und/oder ultravioletten Wellenlängenbereichen aufweisen. Zur Anpassung der von einer LED emittierten Strahlung an den Einsatzzweck werden die LED üblicherweise unterschiedlich dotiert. Zur Emission von UV-Strahlung beispielsweise kommen etwa Diamant, Aluminiumnitrid, Aluminiumgalliumnitrid oder Aluminiumgalliumindiumnitrid als Dotierung in Frage.

Nach einer weiteren Ausführungsform des erfindungsgemäßen Flächenstrahlers kann der Gehäusekörper eine von der Vorderseite abgewandte Rückseite aufweisen und wird zwischen Vorderseite und Rückseite durch Seitenflächen begrenzt. Die Zulauföffnung und die Rücklauföffnung des Fluidwegs können gemeinsam an einer der Seitenflächen oder gemeinsam an der Rückseite, oder einzeln an verschiedenen Seitenflächen oder einzeln jeweils an einer der Seitenflächen und der Rückseite angeordnet sein. Die Anordnung von Zulauföffnung und Rücklauföffnung an Rückseite und/oder Seitenflächen des Gehäusekörpers kann in Abhängigkeit vom Einbaukontext des Flächenstrahlers in einer übergeordneten Vorrichtung zur Beleuchtung, insbesondere zur Durchführung einer photochemischen Reaktion oder zur Desinfektion angepasst ausgewählt sein. Der Gehäusekörper kann ferner zur Anordnung des Flächenstrahlers in einer solchen übergeordneten Vorrichtung ausgebildet sein, indem der Gehäusekörper beispielsweise ein oder mehrere Einbauelemente wie Befestigungsbohrungen, Laschen oder Kragen haben kann.

Eine weitere vorteilhafte Ausführungsform des erfindungsgemäßen Flächenstrahlers sieht vor, dass die Mündungsöffnung an einer der Zulauföffnung abgewandten, d. h. entfernt liegenden Seite der Befestigungsfläche ausgebildet ist. Der erste Kühlkanal verläuft dabei zumindest teilweise in einer Ebene parallel zu der Befestigungsfläche durch den Gehäusekörper, um in einem von der Zulauföffnung entfernten Bereich in den Emissionsraum zu münden. Dadurch, dass sich der erste Kühlkanal unter der Befestigungsfläche durch den Gehäusekörper erstreckt, kann Wärme, die von den LEDs erzeugt und über die Befestigungsfläche von dem Gehäusekörper aufgenommen wird, von dem Gehäusekörper an das zugeführte Kühlmittel bereits bei der Passage des ersten Kühlkanals abgegeben werden. Der Gehäusekörper ist vorzugsweise aus einem wärmeleitenden Material, besonders bevorzugt Aluminium, gefertigt. Auf diese Weise kann auch die Temperatur des Gehäusekörpers gleichbleibend gehalten werden, sodass die von den LEDs erzeugte Wärme nicht nur an der Vorderseite durch den direkten Kontakt der LED mit dem Kühlmittel, sondern auch an der Rückseite durch den Gehäusekörper zu dem Kühlmittel abgeleitet werden kann. Die zumindest eine Ablauföffnung kann vorzugsweise an einer der Rücklauföffnung nahen bzw. zugewandten Seite der Befestigungsfläche ausgebildet sein, sodass der zweite Kühlkanal, durch den das erwärmte Kühlmittel zur Rücklauföffnung geführt wird, möglichst kurz gehalten werden kann.

In Abhängigkeit der Anzahl und/oder Leistung der LEDs, insbesondere bei größerer Anzahl und/oder höherer Leistung der LEDs, kann in einer weiteren Ausführungsform eines erfindungsgemäßen Flächenstrahlers vorgesehen sein, dass die Zulauföffnung mit einem der Zulauföffnung nahen Verteilerkanalabschnitt fluidisch verbunden ist, von dem sich mehrere erste Kühlkanäle durch den Gehäusekörper und parallel zur Befestigungsfläche zu den jeweiligen Mündungsöffnungen erstrecken. Der zweite Kühlkanal kann sich dann von einem Sammelkanalabschnitt, der mit einer Mehrzahl Ablauföffnungen verbunden ist, zu der Rücklauföffnung erstrecken. Alternativ kann sich der erste Kühlkanal von der Zulauföffnung zu einem von der Zulauföffnung entfernten Verteilerkanalabschnitt erstrecken, an dem mehrere Mündungsöffnungen ausgebildet sind, wobei auch hier der zweite Kühlkanal sich von einem Sammelkanalabschnitt, der mit mehreren Ablauföffnungen verbunden ist, zu der Rücklauföffnung erstreckt. In einer möglichen, weniger bevorzugten Alternative kann der Gehäusekörper mehrere erste Kühlkanäle mit entsprechend mehreren, jeweils zugeordneten Zulauf- und Mündungsöffnungen und mehrere zweite Kühlkanäle mit entsprechend mehreren, jeweils zugeordneten Ablauf- und Rücklauföffnungen aufweisen.

Ein erfindungsgemäßer Flächenstrahler kann nach einer weiteren Ausführungsform einen Halterungsrahmen aufweisen, der, vorzugsweise lösbar, an dem Gehäusekörper zur Halterung des Emissionsfensters angeordnet und entsprechend einem Fensterrahmen dazu ausgebildet ist, die durch das Emissionsfenster bereitgestellte Lichtöffnung für das zumindest eine lichtemittierende Halbleiterbauelement unbedeckt bzw. frei zu lassen. Zur Abdichtung der Anordnung aus Halterahmen und Emissionsfenster am Gehäusekörper kann jeweils eine umlaufende Dichtung (z. B. mittels Dichtungsschnur in einer entsprechenden Dichtungsnut in Halterahmen und Gehäusekörper) zwischen dem Halterungsrahmen und dem Emissionsfenster sowie zwischen dem Emissionsfenster und dem Gehäusekörper angeordnet sein.

Alternativ zur Befestigung des Emissionsfensters mittels Halterungsrahmen kann das Emissionsfenster in einer weiteren Ausführungsform durch eine Klebstoffschicht an dem Gehäusekörper befestigt sein, die gleichzeitig für die Abdichtung sorgt. Vorteilhaft kann hierbei eine nahezu randlose Ausführung des Flächenstrahlers geschaffen werden, die sich besonders gut zur Einbindung in eine übergeordnete Vorrichtung eignen kann. Ferner ist eine Ausführungsform denkbar, die Klebstoffschicht und Halterungsrahmen bei der Anordnung des Emissionsfensters an dem Gehäusekörper kombiniert, wenn etwa das Emissionsfenster durch eine Klebstoffschicht in einem Halterungsrahmen abdichtend fixiert wird, der lösbar am Gehäusekörper angeordnet werden kann, sodass nur eine umlaufende Dichtung zur Abdichtung des Halterungsrahmens bzw. Emissionsfenster am Gehäusekörper erforderlich ist.

Eine weitere Ausführungsform des erfindungsgemäßen Flächenstrahlers bezieht sich darauf, dass der Gehäusekörper an der Vorderseite zumindest einen Befestigungsabsatz aufweist, der die Befestigungsfläche als abgestufter umlaufender Rand umgibt, wobei ein erster Befestigungsabsatz zur Aufnahme des Emissionsfensters, d. h. als Auflager für das Emissionsfenster, ausgebildet ist. Damit wird eine die Wegstrecke für die elektromagnetische Strahlung durch das Kühlmittel durch die Höhe des Emissionsraums durch die Beabstandung des ersten Befestigungsabsatzes, auf dem das Emissionsfenster aufliegt, von der Befestigungsfläche definiert. Eine Ausführungsform mit einem zweiten Befestigungsabsatz, der den ersten Befestigungsabsatz als abgestufter umlaufender Rand umgibt, kann zur Anordnung eines Halterungsrahmens zur Befestigung des Emissionsfensters am Gehäusekörper vorgesehen sein.

In einer bevorzugten Ausführungsform kann die Anschlussöffnung über eine Durchtrittsöffnung mit der Anschlusskammer verbunden sein, wobei eine Querschnittfläche der Durchtrittsöffnung kleiner ist als eine Querschnittfläche der Anschlussöffnung, um eine Abdichtung der Anschlussöffnung gegenüber der mit dem Kühlmittel gefüllten Anschlusskammer zu erleichtern.

Die Durchtrittsöffnung kann dabei eine von der Kreisform abweichende Querschnittsform beispielsweise eine polygonale Form haben, oder beispielsweise als Zweiflach ausgebildet sein, um eine rotationsgehinderte Anordnung eines entsprechend geformten Anschlusselements, wie z. B. eines Anschlusssteckers, der mit den Anschlussleitungen verbunden ist, oder eine Adapterelements, durch das sich die Anschlussleitungen erstrecken, zu ermöglichen.

So kann ein erfindungsgemäßer Flächenstrahler in weiteren Ausführungsform zum elektrischen Anschluss vorsehen, dass sich die zumindest eine Anschlussleitung durch die Durchtrittöffnung aus der Anschlusskammer zumindest teilweise in die Anschlussöffnung erstreckt, oder dass der Flächenstrahler einen Anschlussstecker aufweist, der mit der zumindest einen Anschlussleitung (ggf. über ein Vorschaltgerät) verbunden ist, wobei der Anschlussstecker sich zumindest teilweise in die Anschlussöffnung, ggf. auch in die zur Anschlusskammer führende Durchtrittöffnung, erstreckt. Zur Vermeidung von Leckagen durch eine Isoliermantelung der Anschlussleitungen oder des Anschlusssteckers kann die Anordnung des Anschlusssteckers (oder der Anschlussleitungen) in der Anschlussöffnung (und/oder in der zur Anschlusskammer führenden Durchtrittöffnung) durch eine Verguss- oder Lotmasse abgedichtet sein.

Ferner kann nach einer Ausführungsform eines erfindungsgemäßen Flächenstrahlers der Flächenstrahler ein Zulaufanschlusselement, das zum Anschluss einer Kühlmittelzuleitung mit der Zulauföffnung verbunden ist haben. Der Flächenstrahler weist ferner ein Rücklaufanschlusselement auf, das zum Anschluss einer Kühlmittelrückleitung mit der Rücklauföffnung verbunden ist. Dabei ist die Verbindung des Zulaufanschlusselements mit der Zulauföffnung und/oder des Rücklaufanschlusselements mit der Rücklauföffnung zur Vermeidung von Leckagen durch eine Verguss- oder Lotmasse abgedichtet.

Eine erfindungsgemäße Vorrichtung zur Beleuchtung, zur Durchführung einer photochemischen Reaktion oder zur Desinfektion weist zumindest einen Flächenstrahler in einer erfindungsgemäßen Ausführungsform mit zumindest einem lichtemittierenden Halbleiterbauelement auf, dessen Emissionsspektrum eine entsprechende Einsatzstrahlung zur Beleuchtung, zur Durchführung einer photochemischen Reaktion oder zur Desinfektion bereitstellt.

Nach einer weiteren Ausführungsform einer erfindungsgemäßen Vorrichtung zur Beleuchtung, zur Durchführung einer photochemischen Reaktion oder zur Desinfektion, weist die Vorrichtung ein Gehäuse auf. Darunter werden nicht nur eine Wandung, die einen Reaktionsraum vollständig umgibt, sondern auch Halterungen im allgemeineren Sinn verstanden. Somit umgibt das Gehäuse einen Beleuchtungsraum, Reaktionsraum oder Desinfektionsraum zumindest teilweise und weist zumindest einen Einbauplatz für den zumindest einen Flächenstrahler auf. Vorzugsweise weist der Gehäusekörper des Flächenstrahlers zur Anordnung in der Vorrichtung an dem vorbestimmten Einbauplatz zumindest ein Einbauelement auf.

Eine erfindungsgemäße Verwendung eines erfindungsgemäßen Flächenstrahlers ist die Beleuchtung, Durchführung einer photochemischen Reaktion oder Desinfektion, wobei das zumindest eine lichtemittierende Halbleiterbauelement des Flächenstrahlers ein Emissionsspektrum aufweist, das eine entsprechende Einsatzstrahlung zur Beleuchtung, zur Durchführung einer photochemischen Reaktion oder zur Desinfektion bereitstellt.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig.** 1: eine perspektivische Ansicht des Flächenstrahlers nach einer erfindungsgemäßen Ausführungsform,
- **Fig.** 2: eine Frontansicht auf den erfindungsgemäßen Flächenstrahler aus Fig. 1 mit den Schnittlinien AA, CC, DD, EE, FF,
- **Fig. 3**: eine Querschnittansicht durch den Flächenstrahler aus Fig. 2 entlang Schnittlinie AA,
- **Fig. 4**: eine Querschnittansicht durch den Flächenstrahler aus Fig. 2 entlang Schnittlinie CC,
- **Fig. 5**: eine Längsschnittansicht durch den Flächenstrahler aus Fig. 2 entlang Schnittlinie DD,
- **Fig. 6**: eine Längsschnittansicht entsprechend Fig. 5 durch den Gehäusekörper, das Emissionsfenster und den Halterungsrahmen des Flächenstrahlers im demontierten Zustand,
- **Fig. 7**: eine Längsschnittansicht durch den Flächenstrahler aus Fig. 2 entlang Schnittlinie EE,
- **Fig. 8**: eine Längsschnittansicht durch den Flächenstrahler aus Fig. 2 entlang Schnittlinie FF,
- **Fig. 9**: eine schematisch perspektivische Ansicht eines Gehäusekörpers eines erfindungsgemäßen Flächenstrahlers mit dargestelltem Fluidweg a,
- **Fig. 10**: eine Frontansicht auf einen Flächenstrahler nach einer erfindungsgemäßen Ausführungsform mit elektrischen Anschlusselementen,
- **Fig. 11**: eine schematische Längsschnittansicht durch einen Flächenstrahler nach einer weiteren erfindungsgemäßen Ausführungsform,
- **Fig. 12**: eine schematische Frontansicht auf einen Flächenstrahler mit einem mündungsöffnungsnahen Verteilerkanal nach einer weiteren erfindungsgemäßen Ausführungsform,
- **Fig. 13**: eine schematische Frontansicht auf einen Flächenstrahler mit einem mündungsöffnungsfernen Verteilerkanal nach einer weiteren erfindungsgemäßen Ausführungsform,
- **Fig. 14**: eine schematische Frontansicht auf einen Flächenstrahler mit einem mäandrierenden ersten Kühlkanal nach einer weiteren erfindungsgemäßen Ausführungsform,
- **Fig. 15**: eine schematische Querschnittansicht durch eine LED mit Primäroptik, die als lichtemittierendes Halbleiterbauelement in einem erfindungsgemäß ausgeführten Flächenstrahler einsetzbar ist,
- **Fig. 16**: eine schematische Querschnittansicht durch eine primäroptiklose LED, die als lichtemittierendes Halbleiterbauelement in einem Flächenstrahler nach einer weiteren Ausführungsform einsetzbar ist,
- **Fig. 17**: eine schematische Seitenansicht auf eine einen Flächenstrahler aufweisende Vorrichtung nach einer erfindungsgemäßen Ausführungsform zur Durchführung einer photochemischen Reaktion,
- **Fig. 18**: eine schematische Seitenansicht auf eine einen Flächenstrahler aufweisende Vorrichtung nach einer weiteren erfindungsgemäßen Ausführungsform zur Desinfektion.

Die vorliegende Erfindung bezieht sich auf einen LED-Flächenstrahler, der in erster Linie als Hochleistungsstrahler zum industriellen Einsatz vorgesehen ist, beispielsweise als Flächenstrahler in einer Vorrichtung zur Desinfektion von Oberflächen oder zur Durchführung einer photochemischen Reaktion. Dazu kann der Flächenstrahler beispielsweise in einer Halterung oberhalb der zu desinfizierenden Oberfläche oder in einem Wandungselement eines Photoreaktors, das auch ein Reaktordeckel sein kann, montiert werden. Ferner kann ein erfindungsgemäßer LED-Flächenstrahler auch zu Beleuchtungszwecken oder als Wärmestrahler ausgebildet sein, die als Hochleistungsstrahler ebenfalls eine effektive Wärmeableitung erfordern. Entsprechend bezieht sich die vorliegende Erfindung auch auf jedwede Vorrichtung zur Beleuchtung, sowie ggf. auf Vorrichtungen zur Wärmebehandlung, Durchführung einer photochemischen Reaktion oder Desinfektion, die einen Flächenstrahler in einer erfindungsgemäßen Ausführungsform aufweist, sowie auf die Verwendung eines erfindungsgemäßen Flächenstrahlers insbesondere zur Beleuchtung, ggf. aber auch zur Wärmebehandlung, Durchführung einer photochemischen Reaktion oder Desinfektion im allgemeinen.

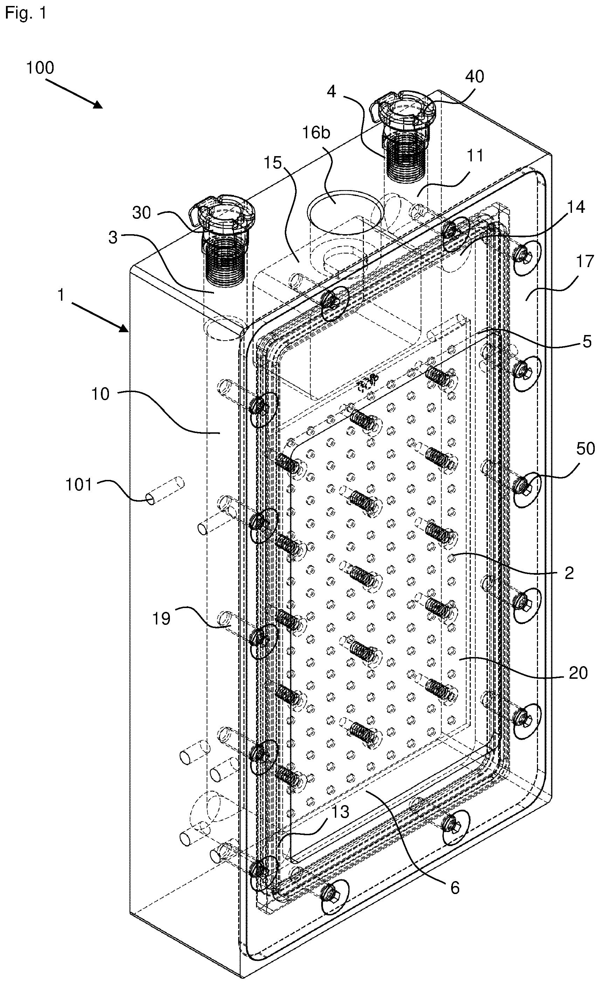

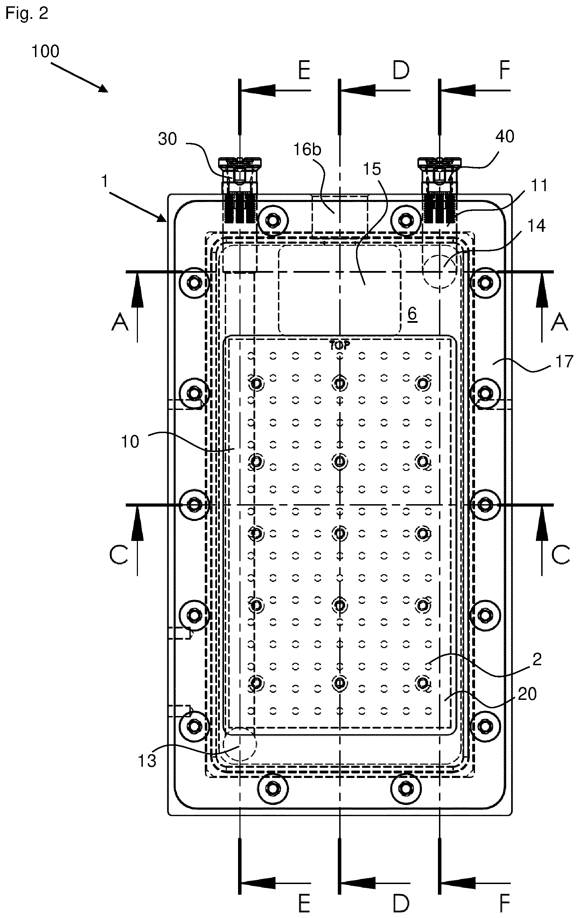

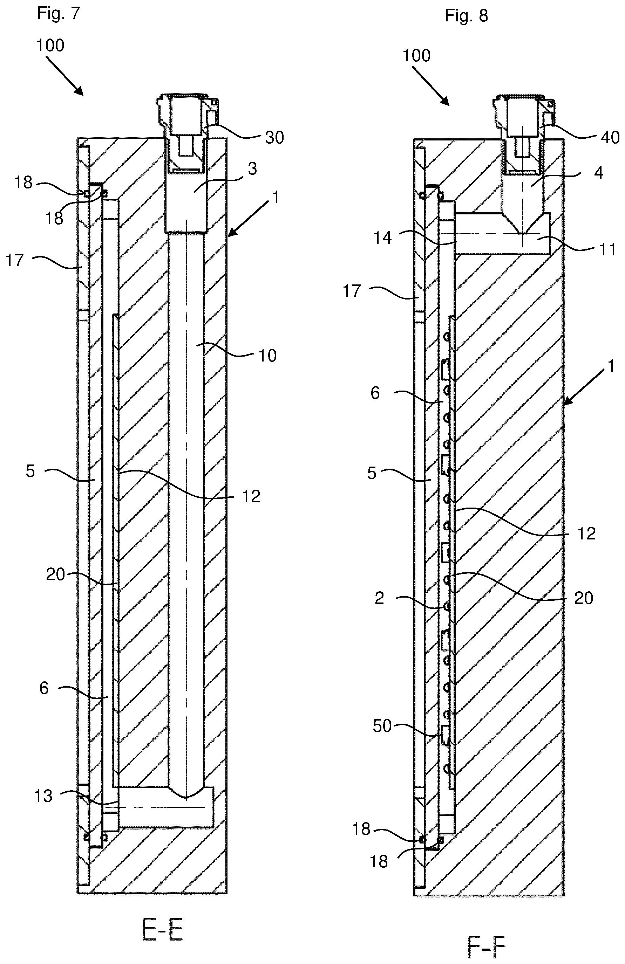

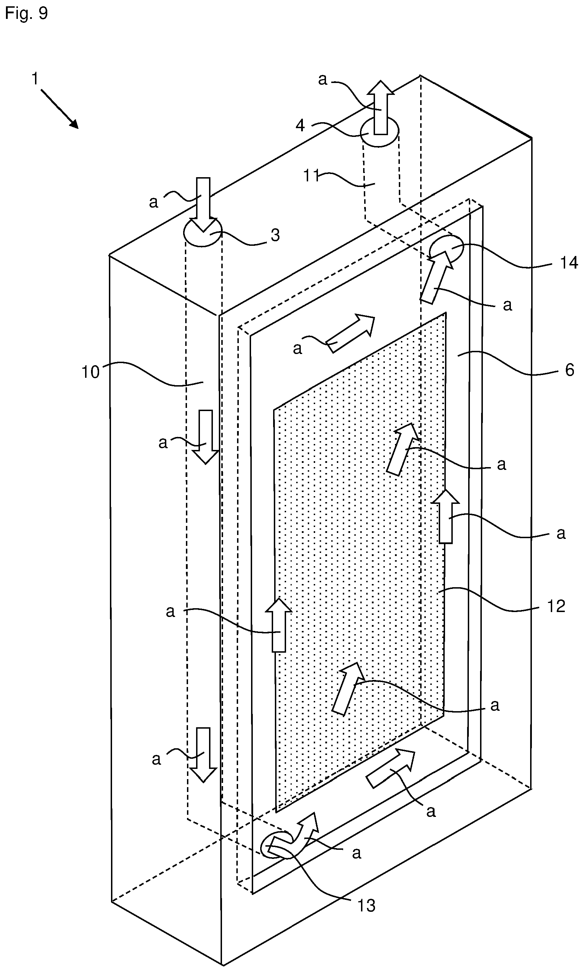

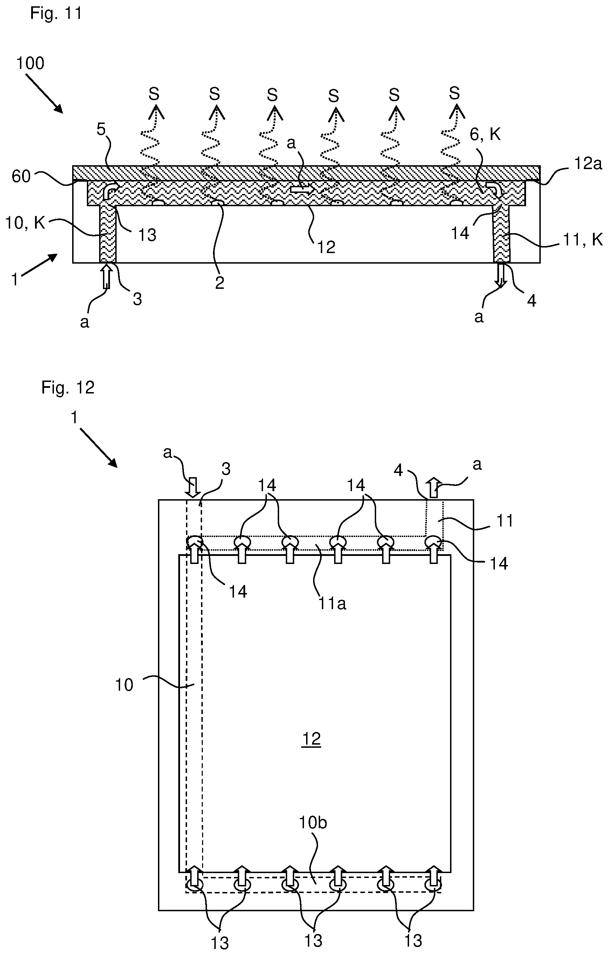

Der Aufbau eines erfindungsgemäßen Flächenstrahlers 100 mit vielen flächig angeordneten LEDs 2 und einem Gehäusekörper 1 wird in **Fig. 1 bis 8** erläutert. **Fig. 9** **und** **11** mit ihren vereinfachten, detailarmen schematischen Darstellungen zeigen das Prinzip der Vorrichtung und insbesondere den Fluidweg a des Kühlmittels K durch einen erfindungsgemäßen Flächenstrahler 100. Dabei hat der Flächenstrahler 100 in **Fig. 11** eine abweichende Kühlkanalgestaltung. In dem Flächenstrahler 100 werden die an einer Befestigungsfläche 12 des Gehäusekörpers 1 angeordneten LEDs 2 von einem Emissionsfenster 5 überlagert, das beabstandet von der Befestigungsfläche 12 an dem Gehäusekörper 1 angeordnet und für eine von den LEDs 2 emittierte Einsatzstrahlung S transparent ist, die in **Fig. 11** durch die gepunkteten Wellenpfeile angedeutet ist. Die Anordnung des Emissionsfensters 5 an dem Gehäusekörper 1 ist dabei fluiddicht ausgebildet, sodass der Gehäusekörper 1, die an der Befestigungsfläche 12 angeordneten LEDs 2 und das Emissionsfenster 5, das eine Vorderseite des Flächenstrahlers 100 definiert, einen Emissionsraum 6 begrenzen, durch den der Fluidweg a verläuft. Der Emissionsraum 6 ist so mit dem Kühlmittel K komplett gefüllt, das die LED 2 somit direkt zur Aufnahme und Ableitung der erzeugten Wärme kontaktiert. Als Kühlmittel K wird daher eine wärmeleitende und elektrisch isolierende Flüssigkeit ausgewählt, die wie das Emissionsfenster 5 für die Einsatzstrahlung S transparent ist. Damit verläuft in einem erfindungsgemäßen Flächenstrahler 100 der Fluidweg a in Strömungsrichtung des Kühlmittels K, ausgehend von einer Zulauföffnung 3, die an einer Außenwand des Gehäusekörpers 1 ausgebildet ist, durch einen ersten Kühlkanal 10, der sich durch den Gehäusekörper 1 zu einer Mündungsöffnung 13 in den Emissionsraum 6 erstreckt, die auf einer ersten Seite benachbart zu der Befestigungsfläche 12 ausgebildet ist. Von der Mündungsöffnung 13 verläuft der Fluidweg a durch den Emissionsraum 6 zu einer Ablauföffnung 14, die auf einer von der ersten Seite abgewandten zweiten Seite neben der Befestigungsfläche 12 ausgebildet ist und den Emissionsraum 6 mit einem zweiten Kühlkanal 11 verbindet, der durch den Gehäusekörper 1 zu einer Rücklauföffnung 4 an einer Außenwand des Gehäusekörpers 1 führt.

Das den Emissionsraum 6 füllende und durchströmende Kühlmittel K leitet einen wesentlichen Anteil der im Betrieb von den LEDs 2 erzeugte Wärme aufgrund des direkten Kontakts effektiv ab. Zusätzlich kann der Gehäusekörper 1 aus einem wärmeleitenden Material gefertigt sein, das insbesondere ein Metallmaterial und besonders bevorzugt Aluminium oder eine Aluminiumlegierung sein kann, sodass auch ein gewisser Anteil der von den LEDs 2 erzeugten Wärme an der Rückseite der LED 2 auf den Gehäusekörper 1 abgeleitet werden kann.

Bevorzugt kann die durch das Kühlmittel K aufgenommene Wärme nach Ausleitung durch die Rücklauföffnung 4 außerhalb des Emissionsraums 6, bzw. außerhalb des Gehäusekörpers 1 abgekühlt werden und das abgekühlte Kühlmittel K im Kreislauf über die Zulauföffnung 3 wieder zugeführt werden. Der Flächenstrahler 100 kann daher entsprechende bekannte Elemente zur Ausbildung eines Kühlmittelkreislaufs aufweisen. Ferner sorgt das den Emissionsraum 6 füllende und durchströmende Kühlmittel K auch für eine thermische Entkopplung der LED 2 von der an das Emissionsfenster 5 angrenzenden Umgebung, indem das Kühlmittel K über das Emissionsfenster 5 auch Wärme mit der Umgebung austauschen kann. Weiter vorteilhaft sorgt das den Emissionsraum 6 füllende Kühlmittel K für eine Vermeidung von Nahfeldreflexion zwischen LEDs 2 und Emissionsfenster 5 und verhindert das Vorkommen von flüchtigen organischen Kohlenwasserstoffverbindungen (VOCs) im Emissionsraum 6, die die LED 2 schädigen könnten. Ferner wird durch die Füllung des Emissionsraums 6 mit dem Kühlmittel eine explosionsfähige Atmosphäre vermieden. Ein erfindungsgemäßer Flächenstrahler 100 ist daher nicht nur hinsichtlich der Kühlung der LEDs 2 und deren thermischer Abkopplung von der Umgebung verbessert, sondern schützt die LEDs 2 zudem vor schädlichen Substanzen und stellt darüber hinaus besonders vorteilhaft eine erhöhte Gesamtlicht- bzw. Strahlungsleistung der Einsatzstrahlung S in Bezug auf den Stand der Technik bereit. Dies resultiert daraus, dass das flüssige Kühlmittel K, das bevorzugt aus gesättigten Kohlenwasserstoffen, Silikonölen und synthetischen Ester- und Etherverbindungen ausgewählt wird und dessen Brechungsindex deutlich größer als der von Luft oder Inertgas ist und für geeignete Kühlflüssigkeiten im Bereich von etwa 1,35 bis etwa 1,55 (bei 20°C) liegt, die Photonen-Auskopplungseffizienz an der Phasengrenze der Diodenoberfläche zum Kühlmittel K im Emissionsraum 6 vergrößert. Dabei wird die Reflexion an der Phasengrenze zwischen dem Kühlmittel K im Emissionsraum 6 und dem Emissionsfenster 5 verringert.

Die Einsatzstrahlung für einen vorbestimmten Einsatz des Flächenstrahlers, z. B. zur Beleuchtung, Durchführung einer photochemischen Reaktion oder zur Desinfektion wird durch LEDs bereitgestellt, deren Dotierung für elektromagnetische Strahlung mit der/den für den Einsatz vorgesehene(n) Wellenlänge(n) sorgt. Bekanntermaßen stehen LEDs zur Verfügung, die nicht nur das sichtbare Licht-, sondern auch Infrarot- und/oder UV-Spektralbereiche abdecken. Die Materialwahl des Emissionsfensters und des Kühlmittels kann daher durch den Wellenlängenbereich der Einsatzstrahlung beschränkt sein. So ist zur Beleuchtung die Einsatzstrahlung sichtbares Licht, sodass das Emissionsfenster zumindest für den Teil des elektromagnetischen Spektrums mit Wellenlängen von ca. 380 bis 780 nm transparent ist. UV-Strahlung (100-380 nm) kann als Einsatzstrahlung zur Desinfektion und zur Durchführung photochemischer Reaktionen eingesetzt werden, sodass das Emissionsfenster jeweils aus einem zumindest für die eingesetzten UV-Wellenlängen transparenten Material beschaffen ist. So wird zu Desinfektionszwecken UV-C-Strahlung (100-280 nm) eingesetzt. UV-B (280-315 nm) und UV-A-Strahlung (315-380 nm) findet für viele photochemische Reaktionen Anwendung, ohne dass photochemische Reaktionen auf diesen Spektralbereich beschränkt sind, da die für die Durchführung von photochemischen Reaktionen erforderliche Einsatzstrahlung von dem Typ der beabsichtigten Reaktion bzw. einer Absorptionswellenlänge eines Eduktmoleküls abhängt, sodass die Einsatzstrahlung für bestimmte photochemische Reaktionen ggf. auch in den anderen Wellenlängenbereichen liegen kann. Sollen hier Bezugszeichen rein oder alle raus?

Grundsätzlich kann das Emissionsfenster auch für andere Wellenlängen als die der Einsatzstrahlung transparent sein, es kann aber auch - gerade im Bereich photochemischer Reaktionen - wünschenswert sein, dass das Emissionsfenster nur für bestimmte Wellenlängen transparent sein soll, sodass das Emissionsfenster - durch Materialwahl oder eine Beschichtung - auch eine Filterfunktion übernehmen kann, um unerwünschte Wellenlängen herauszufiltern. Die Auswahl des Fenstermaterials, beispielsweise aus verschiedenen Glas- und Kunststoffmaterialien, in Abhängigkeit der Wellenlängen der Einsatzstrahlung ist eine fachübliche Tätigkeit. So weiß der Fachmann, dass z. B. synthetische Quarz- und hochborhaltige Borosilikatgläser auch im UV-C-Bereich noch eine gute Transparenz besitzen. Im infraroten Spektralbereich zeigen ferner Glaskeramiken gute Transparenz und können dort als Fenstermaterial eingesetzt werden. Ist der Einsatzzweck die Durchführung einer photochemischen Reaktion, ist bei der Auswahl eines Fenstermaterials darauf zu achten, dass keine Reaktionen mit den Edukten oder Produkten stattfinden. Entsprechend wird das Kühlmittel aus elektrisch isolierenden Flüssigkeiten in Abhängigkeit des Spektralbereichs der jeweiligen Einsatzstrahlung ausgewählt. Als "transparent" werden Fenstermaterialien und Kühlflüssigkeiten angesehen, wenn sie für die Wellenlänge(n) der Einsatzstrahlung entlang der jeweiligen Wegstrecke durch das Emissionsfenster bzw. durch den Emissionsraum zwischen LED-Oberfläche und Emissionsfenster einen Transmissionsgrad von jeweils zumindest 75 % aufweisen.

Das flüssige Kühlmittel für Einsatzstrahlung im sichtbaren und UV-Spektralbereich kann beispielsweise aus hochraffinierten Mineralölen ausgewählt werden, die praktisch ausschließlich Alkane und Cycloalkane, also gesättigte Kohlenwasserstoffe umfassen. Vorteilhaft sind Alkane und Cycloalkane vom sichtbaren Wellenlängenbereich bis in den weiten UV-C-Bereich (220-230 nm) transparent. Darunter nimmt die Transmission ab, kann aber, insbesondere bei ausreichend geringer Wegstrecke zwischen LED und Emissionsfenster, noch ausreichend für Wellenlängen bis 195 nm und darunter sein. Cycloalkane können wegen des höheren Brechungsindex' im Vergleich zum entsprechenden linearen Alkan bevorzugt sein. So erstrecken sich die Brechungsindices (20°C) für C₅-C₁₄ Cycloalkane über einen Bereich von etwa 1,41 bis 1,55, während die Brechungsindices (20°C) für die korrespondierenden linearen C₅-C₁₄ Alkane einen Bereich von etwa 1,36 bis etwa 1,43 überdecken. Cyclohexan beispielsweise hat einen Brechungsindex von etwa 1,43, während Hexan einen Brechungsindex von etwa 1,37 aufweist. Ein Nachteil der gesättigten Kohlenwasserstoffe ist vor allem die Bildung leicht entzündlicher Dampf-Luft-Gemische mit einer Einordnung in Temperaturklasse 3, die für den Betrieb in entzündlichen Atmosphären eine maximale Oberflächentemperatur von 200 °C festlegt. Daher ist bei Verwendung von hochraffinierten Mineralölen als Kühlmittel auf einen sorgfältigen und abgedichteten Luftabschluss zu achten, um die Bildung solcher entzündlicher Dampf-Luft-Gemische zu vermeiden.

Eine bevorzugte erfindungsgemäße Ausführungsform kann als Kühlmittel für Einsatzstrahlung im sichtbaren und UV-Spektralbereich dünnflüssige Silikonöle vorsehen, die Brechungsindices im Bereich von etwa 1,37 bis 1,40 aufweisen, vorteilhaft nicht brennbar sind und die vom sichtbaren Wellenlängenbereich bis in den mittleren UV-C-Bereich (etwa 250 nm) transparent sind. Unterhalb von 250 nm beginnt allerdings die Transmission abzunehmen und Wellenlängen kleiner 200 nm werden absorbiert, sodass Silikonöle vor allem für Einsatzgebiete, die Wellenlängen größer 250 nm nutzen wollen, geeignet sind. Für Einsätze, die Wellenlängen im Bereich von 200 bis 250 nm nutzen wollen, eignen sich Silikonöle nur bedingt, nämlich wenn die Wegstrecke im Emissionsraum zwischen LED und Emissionsfenster und damit die Absorption klein genug ist, um eine ausreichende Transmission zu gestatten. Andernfalls sollte im Spektralbereich unter 250 nm auf gesättigte Kohlenwasserstoffe als Kühlmittel zurückgegriffen werden.

Weitere alternative Beispiele für erfindungsgemäß einsetzbare Kühlmittel für Einsatzstrahlung im sichtbaren und UV-Spektralbereich umfassen synthetische Ester- und Etherverbindungen. Synthetische organische Esteröle haben gegenüber den Mineralölen den Vorteil u. a. einer höheren Temperaturbeständigkeit und höheren Brenn- und Zündtemperatur und sind umweltverträglicher, weisen aber eine geringere Alterungsbeständigkeit auf und sind bis in den mittleren UV-Bereich (etwa 270 bis 280 nm) transparent, darunter nimmt die Absorption deutlich zu. Auch bei Etherverbindungen wie beispielsweise 1,4-Dioxan mit einem Brechungsindex von 1,422 reicht die Transmission bis in den mittleren UV-Bereich (270 bis 300 nm, abgesehen von Diethylether bis 255 nm), allerdings nimmt die Transmission darunter weniger steil ab, sodass bei ausreichend geringer Wegstrecke zwischen LED und Emissionsfenster Etherverbindungen als Kühlmittel auch für Wellenlängen unter 270 nm eingesetzt werden können. Wellenlängen kleiner 220 nm werden allerdings absorbiert. Hinsichtlich der Sicherheitstechnik ist aber zu berücksichtigen, dass Etherverbindungen leicht entzündliche Dampf-Luft-Gemische bilden, wobei große Unterschiede zwischen den verschiedenen Etherverbindungen bestehen. Diethylether beispielsweise fällt unter Temperaturklasse T4 (maximal zulässige Oberflächentemperatur 135 °C), während 1,4-Dioxan unter Temperaturklasse 2 (maximal zulässige Oberflächentemperatur 300 °C) fällt, sodass eher 1,4-Dioxan als Kühlmittel eingesetzt werden kann.

Je nach Wellenlänge der Einsatzstrahlung können ggf. auch fluorierte Kohlenwasserstoffe wie Perfluorkohlenwasserstoffe und Hydrofluorether als Kühlmittel eingesetzt werden, die vorteilhaft nicht brennbar sind, aber in gewissen Wellenlängenbereichen Absorptionsbanden aufweisen: Liegt die Einsatzstrahlung außerhalb der Absorptionsbanden können fluorierte Kohlenwasserstoffe wie beispielsweise 3M Fluorinert Electronic Liquid oder 3M Novec High-Tech Flüssigkeit von 3M^{™} (3M electronics, St. Paul, USA) eingesetzt werden. Selbstverständlich kommen auch weitere Flüssigkeiten zum Einsatz in einem erfindungsgemäßen Flächenstrahler als Kühlmittel in Frage, solange sie elektrisch isolierend sind und für die Wellenlänge der Einsatzstrahlung transparent sind. Um eine für die gewünschte Transparenz erforderliche Transmission von zumindest 75 % bereitzustellen, kann der Abstand des Emissionsfensters von der Befestigungsfläche mit den LEDs möglichst minimiert werden, sodass die Wegstrecke durch den mit Kühlmittel gefüllten Emissionsraum zwischen LED und Emissionsfenster - und damit die Absorption der Einsatzstrahlung entsprechend verringert wird. Gleichzeitig sind bei der Dimensionierung des Emissionsraums in Bezug auf den Abstand des Emissionsfensters von der Befestigungsfläche mit den LEDs die Bedingungen für eine optimale Strömungsführung in Verbindung mit einem ausreichenden Flüssigkeitsvolumen für einen optimalen Wärmeabtransport zu berücksichtigen.

In einer bevorzugten Ausführungsform des erfindungsgemäßen Flächenstrahlers 100 erstreckt sich der erste Kühlkanal 10 in einer Weise durch den Gehäusekörper 1, dass die Mündungsöffnung(en) 13 an einer von der Zulauföffnung 3 entfernten Seite neben der Befestigungsfläche 12 ausgebildet ist, wie in **Fig. 1 bis 10** **und** **12 bis 14** zu sehen ist, sodass der erste Kühlkanal 10 zumindest teilweise in einer Ebene parallel zu der Befestigungsfläche 12 durch den Gehäusekörper 1 verläuft. Auf diese Weise kann das durch den ersten Kühlkanal 10 strömende Kühlmittel K bereits bei der Passage des ersten Kühlkanals 10 einen von den LEDs 2 über den Gehäusekörper 1 abgeleiteten Wärmeanteil aufnehmen, ehe das Kühlmittel K in und durch den Emissionsraum 6 strömt, um den Hauptanteil Wärme von den LEDs 2 durch direkten Kontakt abzuführen. Die Gestaltung des ersten Kühlkanals 10 (Abstand von der Befestigungsfläche 12 und Verlauf unter der Befestigungsfläche 12) und ein angepasster Kühlmitteldurchsatz können dafür sorgen, dass ein Temperaturanstieg des Kühlmittels durch die Passage des ersten Kühlkanals 10 gering ist. Der zur Ablauföffnung 14 führende zweite Kühlkanal 11, durch den das durch die Passage des Emissionsraums 6 erwärmte Kühlmittel K strömt, wird möglichst kurz gehalten, um die Wärmeübertragung an dem Gehäusekörper 1 zu minimieren. Daher ist die Ablauföffnung 14 in den gezeigten Beispielen an einer der Rücklauföffnung 4 nahen Seite der Befestigungsfläche 12 ausgebildet.

In den Beispielen der **Fig. 1 bis 11** weist der jeweilige Flächenstrahler 100 einen ersten Kühlmittelkanal 10, der von einer Zulauföffnung 3 zu einer Mündungsöffnung 13 führt, und einen zweiten Kühlmittelkanal 11 auf, der die Ablauföffnung 14 mit der Rücklauföffnung 4 verbindet. Diese einfache Ausführungsform kann - je nach Größe des Flächenstrahlers 100, d. h. Anzahl und Verteilung der LEDs 2 an der Befestigungsfläche 12 sowie deren Leitung - durchaus ausreichend zur effektiven Kühlung der LED 2 sein. Die Mündungsöffnung 13 und die Ablauföffnung 14 sind dabei nicht nur an entgegengesetzten Seiten der Befestigungsfläche 12 angeordnet, sondern auch diagonalen Ecken der Befestigungsfläche 12 zugeordnet, um an möglichst allen LEDs 2 möglichst gleichmäßig vorbei zu strömen.

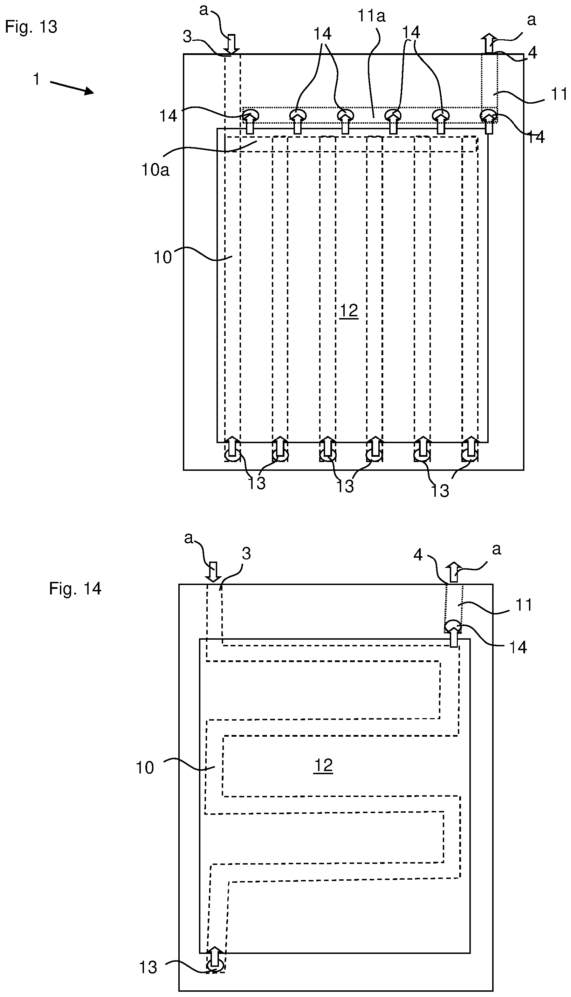

Zur weiteren Vergleichmäßigung der Kühlmittelströmung durch den Emissionsraum 6 können an den voneinander abgewandten Seiten der Befestigungsfläche 12 jeweils mehrere Mündungs- und Ablauföffnungen 13, 14 vorgesehen werden, die entlang der jeweiligen Seite gleichmäßig verteilt angeordnet sind, wie **Fig. 12** **und** **13** zeigen. Die schematischen Darstellungen sollen nur das jeweilige Prinzip verdeutlichen, sind aber nicht maßstabsgerecht, und stellen keine Beschränkung hinsichtlich Anzahl und Anordnung der Öffnungen sowie Verlauf und Durchmesserverhältnisse der Kanäle dar. Fig. 12 verdeutlicht eine Variante, bei der der erste Kühlkanal 10 durch den Gehäusekörper 1 von der Zulauföffnung 3 zu einem Verteilerkanalabschnitt 10b verläuft, der sich in dem Gehäusekörper 1 bezogen auf die Befestigungsfläche 12 entlang einer Seite erstreckt, die von dem Zulauf 3 entfernt bzw. abgewandt liegt. Der Verteilerkanalabschnitt 10b ist mit einer Mehrzahl Mündungsöffnungen 13 verbunden, die auf der zulauffernen Seite neben der Befestigungsfläche 12 ausgebildet sind. Auf der anderen, zulaufnahen Seite der Befestigungsfläche 12 ist eine entsprechende Mehrzahl Ablauföffnungen 14 ausgebildet, die über einen Sammelkanalabschnitt 11a mit dem zweiten Kühlkanal 11 verbunden sind, der sich zu der Rücklauföffnung 4 erstreckt.

In einer modifizierten, nicht dargestellten Variante kann der Gehäusekörper 1 mehrere parallele erste Kühlkanäle 10 aufweisen, von denen sich jeder von jeweils einer zugeordneten Zulauföffnung 3 zu einer entsprechend zugeordneten Mündungsöffnung 13 erstreckt. Entsprechend kann der Gehäusekörper 1 mehrere zweite Kühlkanäle 11 aufweisen, von denen sich jeder von jeweils einer zugeordneten Ablauföffnung 14 zu jeweils einer zugeordneten Rücklauföffnung 4 erstreckt. Diese Ausführungsform ist weniger bevorzugt, da mehrere Zulauf- und Rücklaufanschlüsse für das Kühlmittel erforderlich sind. Dennoch kann eine Gestaltung des Fluidpfads a mit den mehreren Kühlkanäle für gewisse Ausführungsformen des Flächenstrahlers 100 in Abhängigkeit der Anzahl, Anordnung und Leistung der verwendeten LED 2 sinnvoll sein.

Fig. 13 zeigt eine Gestaltung des Fluidpfads a ähnlich wie in Fig. 12, allerdings mit dem Unterschied, dass der Verteilerkanalabschnitt 10a hier in Bezug auf die Befestigungsfläche 12 an einer zulaufnahen, d. h. mündungsöffnungsfernen Seite ausgebildet ist, sodass sich mehrere erste Kühlkanäle 10 von dem Verteilerkanalabschnitt 10a durch den Gehäusekörper 1 parallel zur Befestigungsfläche 12 zu den jeweils zugeordneten Mündungsöffnungen 13 erstrecken. Mit der Anordnung mehrerer erster Kühlkanäle 10, die sich unter der Befestigungsfläche 12 parallel dazu zu den Mündungsöffnungen 13 erstrecken, kann, falls erforderlich, eine gleichmäßigere Temperierung des Gehäusekörpers 1 bei der Passage der ersten Kühlkanäle durch das Kühlmittel erzielt werden als bei einem einzigen ersten Kühlkanal 10, der geradlinig einseitig unter der Befestigungsfläche 12 verläuft. Allerdings kann auch mit nur einem Kühlkanal 10 eine gleichmäßigere Temperierung erreicht werden, wenn dieser einen die Befestigungsfläche 12 gleichmäßig abdeckenden Verlauf aufweist, wie beispielsweise den mäandrierenden Verlauf in **Fig. 14****.** Während die einfacheren Kühlkanalgestaltungen der Beispiele aus **Fig. 1 bis 11** mit maximal einem Richtungswechsel der Kühlkanalabschnitte durch ein- oder zweiseitige Bohrungen in den Gehäusekörper 1 gefertigt werden können, erfordern die komplexeren Kühlkanalgestaltungen mit mehr als einem Richtungswechsel der Kanalabschnitte mehrseitige Bohrungen mit nachfolgenden Verschlüssen der Bohrungsabschnitte, die nicht zu dem Fluidpfad gehören, oder ggf. ein generatives Fertigungsverfahren des Gehäusekörpers.

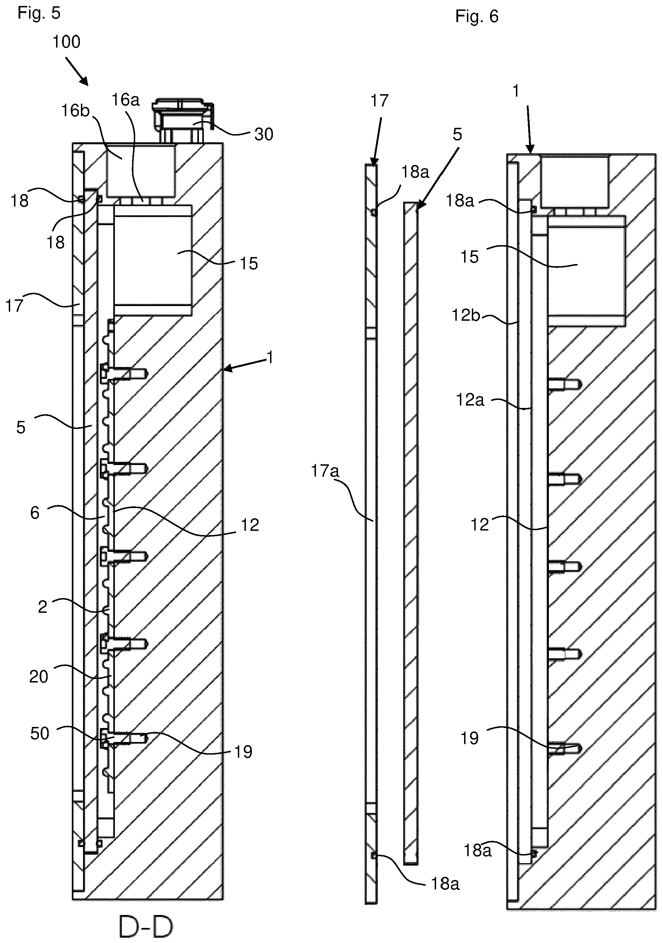

Zur fluiddichten Anordnung des Emissionsfensters 5 an dem Gehäusekörper 1, um den mit Kühlmittel K gefüllten Emissionsraum 6 gegen die Umgebung abzudichten, ist bei dem in **Fig. 1 bis 10** gezeigten beispielhaften Flächenstrahler 100 ein Halterungsrahmen 17 vorgesehen, der in diesem Beispiel mittels Schrauben 50, für die sowohl im Halterungsrahmen 17 als auch im Gehäusekörper 1 entsprechende Befestigungsbohrungen 19 vorgesehen sind, lösbar an dem Gehäusekörper 1 befestigt ist. Der Halterungsrahmen 17 begrenzt eine Öffnung 17a, die in ihre Flächendimensionierung im Wesentlichen der Befestigungsfläche 12 entspricht, um die LED 2 unbedeckt zu lassen und den Strahlungsaustritt durch das Emissionsfenster 5 zu gestatten. Die Abdichtung des Emissionsraums 6 erfolgt hierbei durch die Anordnung jeweils einer umlaufenden Dichtung 18 zwischen dem Halterungsrahmen 17 und dem Emissionsfenster 5 sowie zwischen dem Emissionsfenster 5 und dem Gehäusekörper 1, wofür der Halterungsrahmen 17 und der Gehäusekörper 1 jeweils entsprechende Dichtungsnuten 18a (bezeichnet in **Fig. 6****)** zur Aufnahme einer Dichtungsschnur oder eines entsprechenden Dichtungselements aufweisen.

Der erfindungsgemäße Flächenstrahler 100 aus dem Beispiel der **Fig. 11** verzichtet auf einen Halterungsrahmen, indem das Emissionsfenster 5 durch eine Klebstoffschicht 60 an dem Gehäusekörper 1 befestigt und gleichzeitig abgedichtet ist. Damit ist der Flächenstrahler 100 nahezu randlos ausgeführt und damit besonders geeignet zum nahtlosen Einbau in ein Reaktorwandelement einer Vorrichtung zur Durchführung einer photochemischen Reaktion zum Beispiel.

Selbstverständlich sind auch Variationen und Kombinationen der Befestigung und Abdichtung eines Emissionsfensters an einem Gehäusekörper denkbar, wie z. B. dass ein Emissionsfenster durch eine Klebstoffschicht an einem Halterungsrahmen befestigt und abgedichtet wird, der lösbar am Gehäusekörper befestigt ist, wobei die Anordnung des Halterungsrahmen am Gehäusekörper durch ein Dichtmittel abgedichtet ist.

Ist ein Einsatz eines erfindungsgemäßen Flächenstrahlers in einer Vorrichtung zur Durchführung einer photochemischen Reaktion vorgesehen, so ist die Abdichtung des Emissionsfensters an dem Gehäusekörper hinsichtlich Materialwahl und Formgebung entsprechend den vorgesehenen Reaktionsdrücken und Reaktionstemperaturen ausgelegt, die in dem an das Emissionsfenster angrenzenden Reaktionsraum herrschen können. Hierzu zählen insbesondere von Raumtemperatur und Umgebungsdruck abweichende Reaktionstemperaturen und -drücke, die in dem an das Emissionsfenster angrenzenden Reaktionsraum herrschen und auch Temperaturen unter + 5 °C und über + 40 °C sowie Drücke im Bereich von Hochvakuum und 6 bar Überdruck umfassen können.

Wie besonders gut in **Fig. 6** zu sehen, weist der Gehäusekörper 1 der gezeigten beispielhaften Ausführungsform eines erfindungsgemäßen Flächenstrahlers 100 zur Anordnung des Emissionsfensters 5 einen ersten Befestigungsabsatz 12a und zur Anordnung des Halterungsrahmens 17 einen zweiten Befestigungsabsatz 12b auf, die stufenweise die Befestigungsfläche 12 umgeben. Der erste Befestigungsabsatze 12a wird dabei durch die Abstufung des zweiten Befestigungsabsatzes 12b umrandet, wobei die Abmessungen der umrandeten Fläche dem anzuordnenden Emissionsfenster 5 und die Höhe der Abstufung zum zweiten Absatz 12b der Dicke des Emissionsfensters 5 entsprechen. Die Höhe der Abstufung des ersten Befestigungsabsatzes 12a in Bezug auf die Befestigungsfläche 12 bestimmt den Abstand des Emissionsfensters 5 von der Befestigungsfläche 12 und damit die Höhe des Emissionsraums 6, die die Wegstrecke für die von den LEDs 2 emittierte Strahlung durch das Kühlmittel K definiert. Auch der zweite Befestigungsabsatz 12b für den Halterungsrahmen 17 wird durch einen Umrandungssteg begrenzt, wobei die Abmessungen der hierdurch umrandeten Fläche und die Höhe des Umrandungsstegs den jeweiligen Abmessungen des anzuordnenden Halterungsrahmens 17 entsprechen.

Auch der Gehäusekörper 1 des Beispiels aus **Fig. 11****,** bei dem das Emissionsfenster 5 an dem Gehäusekörper 1 durch eine Klebstoffschicht 60 befestigt wird, weist einen ersten Befestigungsabsatz 12a auf, allerdings ohne Umrandung durch einen weiteren Befestigungsabsatz oder Steg, sodass das Emissionsfenster 5 die Vorderseite des Gehäusekörpers 1 flächig bedeckt. Abweichend von dem dargestellten Beispiel kann auch zur Anordnung eines mittels Klebschicht befestigten Emissionsfenster eine Umrandung des ersten Befestigungsabsatzes 12a durch einen weiteren Absatz oder Steg vorgesehen sein, wobei die Abmessungen der hierdurch umrandeten Fläche und eine Höhe des Umrandungsstegs den jeweiligen Abmessungen des Emissionsfensters entsprechen.

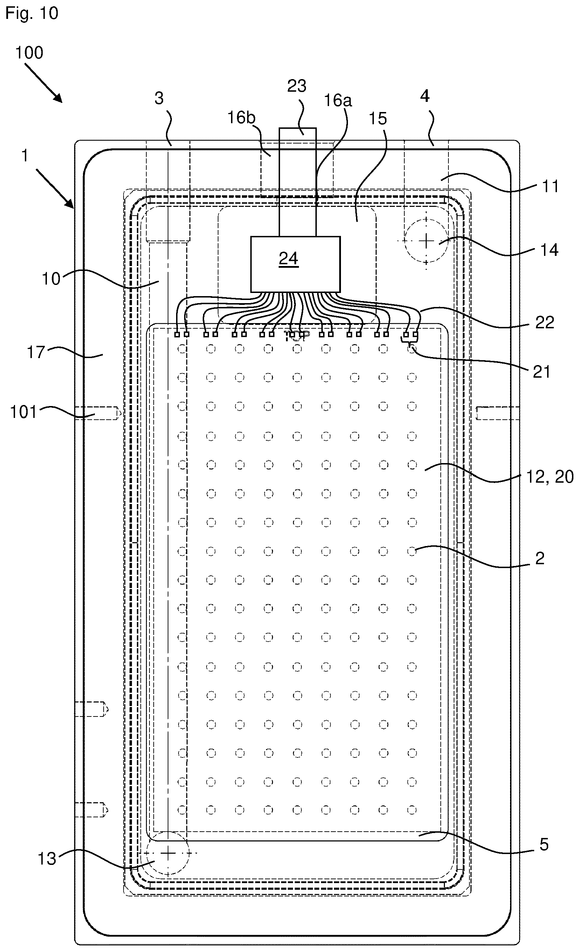

Zur Unterbringung von elektrischen Anschlusselementen für die LEDs 2 weist der erfindungsgemäße Flächenstrahler 100 der Beispiele aus **Fig. 1 bis 8** **und** **10** an der Vorderseite in dem Gehäusekörper 1 benachbart zu der Befestigungsfläche 12 eine Anschlusskammer 15 auf, die zu dem Emissionsraum 5 geöffnet in dem Gehäusekörper 1 zwischen dem ersten und dem zweiten Kühlkanal 10, 11 ausgebildet ist. Die Anschlusskammer 15 wird im dargestellten Beispiel durch den Halterungsrahmen 17 verdeckt, der dazu auf der entsprechenden Seite einen breiteren Rahmenabschnitt aufweist. Mit der Anschlusskammer 15 ist über eine Durchtrittöffnung 16a eine Anschlussöffnung 16b in dem Gehäusekörper 1 ausgebildet, die einen elektrischen Anschluss an eine Energiequelle gestattet. Die elektrischen Anschlusselemente, die sich in die Anschlusskammer 15 erstrecken, umfassen in dem in **Fig. 10** dargestellten Beispiel Anschlussleitungen 22 zum Anschluss der LEDs 2.

Bei dem dargestellten beispielhaften Flächenstrahler 100 sind die lichtemittierenden Halbleiterbauelemente 2 auf einer Platine 20 angeordnet, die zum Anschluss der LEDs 2 entsprechende Leiterbahnen (nicht dargestellt) aufweist, die zu Anschlusskontakten 21 führen, die an einer der Anschlusskammer 15 nahen Seite der Platine 20 angeordnet sind. Jeweils ein Paar Anschlusskontakte 21 ist für jede Reihe LED 2 vorgesehen und mit einer jeweiligen Anschlussleitung 22 verbunden. Die Platine 20 kann, wie im Beispiel von **Fig. 1 bis 8** mittels Schrauben 50 an der Befestigungsfläche 12 des Gehäusekörpers 1 befestigt sein, der dazu entsprechende Befestigungsbohrungen 19 aufweist.

Die LEDs 2 können allerdings, wie in **Fig. 11** skizziert, auch einzeln ohne Platine 20 an der Befestigungsfläche 12 befestigt werden. Dann kann jede LED 2 einen eigenen Anschluss aufweisen, wobei eine solche Ausführung insbesondere für einen Flächenstrahler 100 mit einer begrenzten Anzahl an LEDs oder für einen Flächenstrahler 100 in Frage kommt, bei dem die LEDs in einer einzigen Reihe angeordnet sind, die einen seitlichen Anschluss jeder LED erlaubt. Bei einem Flächenstrahler mit mehreren Reihen LEDs kann bei Verzicht auf eine Platine 20, die Leiterbahnen zum elektrischen Anschluss der LEDs aufweist, beispielsweise vorgesehen sein, dass die Befestigungsfläche Schlitze aufweist.

Die Schlitze sind so in die Befestigungsfläche eingebracht, dass darin Anschlussleitungen zu den jeweiligen LEDs verlaufen können und so keine Verschattungen der LEDs durch die Anschlussleitungen auftreten. Der individuelle Anschluss der LEDs, die somit einzeln ansteuerbar sind, ermöglicht, dass bei Ausfall einer LED nur die betroffene und nicht alle LEDs abgeschaltet werden müssen, sodass die restlichen LEDs weiter in Betrieb bleiben.

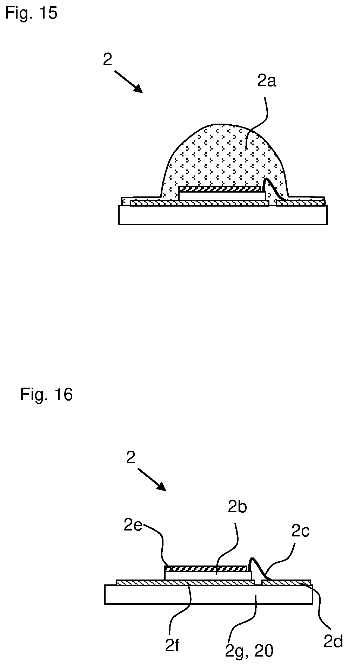

In **Fig. 15** ist a) eine herkömmliche LED 2 mit einer Kunststofflinse als Primäroptik 2a dargestellt, die in einem erfindungsgemäßen LED-Flächenstrahler 100 zum Einsatz kommen kann. Vorteilhaft ist durch die Flutung mit Kühlmittel im Gegensatz zur Verwendung von einem Inertgas wie Stickstoff, dass eine beschleunigte Alterung der Primäroptiken 2a der LED 2 vermieden wird, da gerade in Chemieanlagen VOCs ("volatile organic compounds") vorhanden sind, die in die üblicherweise als Silikonlinse ausgeführten Primäroptiken 2a eindringen, diese eintrüben und somit die Lichtausbeute senken. Da die Primäroptiken 2a erfindungsgemäß keiner gashaltigen Atmosphäre mehr ausgesetzt sind, sondern durch das Kühlmittel abgeschirmt werden, wird der Alterungsprozess deutlich verlangsamt. Um die Einschränkungen in der Lichtausbeute infolge des Alterungsprozesses der Primäroptiken 2a gänzlich zu vermeiden, besteht bei einem erfindungsgemäßen Flächenstrahler 100 vorteilhaft die Möglichkeit, dass die LED 2 des Flächenstrahlers 100 gänzlich auf Primäroptiken 2a wie Silikonlinsen verzichten, da durch das flüssige Kühlmittel der Halbleiterchip der LED bereits ausreichend vor Umwelteinflüssen geschützt ist und das Kühlmittel die Funktionen der Primäroptik übernimmt. **Fig. 16** zeigt eine primäroptiklose LED 2, deren Aufbau ansonsten mit Halbleiterkristall 2b, Draht 2c, Anode 2d, LED-Chip 2e, Kathode 2f und Basisstruktur 2g bzw. Platine 20 der herkömmlichen LED 2 aus **Fig. 15** entsprechen kann, bei der vorteilhaft, abgesehen von der Einsparung eines Bauteils, der Kühleffekt weiter verbessert ist.

In der Anschlusskammer 15 kann ferner, wie in **Fig. 10** zu sehen, ein Vorschaltgerät 24 für die LEDs 2 untergebracht sein, das mit den Anschlussleitung 22 verbunden ist. Aufgrund der fluiden Verbindung der Anschlusskammer 15 mit dem Emissionsraum 6 ist die Anschlusskammer 15 ebenfalls mit Kühlmittel gefüllt und kann die im Betrieb vom Vorschaltgerät 24 erzeugte Wärme mit abführen. Damit kann auf eine Anordnung des Vorschaltgeräts 24 auf der Platine 20 verzichtet werden, wodurch ein Wärmeeintrag durch das Vorschaltgerät 24 über die Platine 20 auf die benachbarten LEDs 2 vermieden wird. Eine Anordnung eines Vorschaltgeräts 24 auf einer Platine 20 in einem erfindungsgemäßen Flächenstrahler 100 ist allerdings nicht ausgeschlossen, da auch in einer solchen Ausführungsform das Kühlmittel für eine effektive Wärmeabfuhr sorgt. Ferner können in einer nicht dargestellten Alternative Vorschaltgeräte wie Gleichspannungswandler oder auch Netzteile in einem Gehäuseaufsatz rückseitig auf dem Gehäusekörper montiert werden. Auch hier kann das flüssige Kühlmittel zur Kühlung der Gleichspannungswandler oder andere Vorschaltgeräte durch Anschluss des Gehäuseaufsatzes an den Kühlkreis verwendet werden. Zum sicheren Betrieb mit ATEX-Zertifizierung kann eine Inertisierung, ein druckfeste Auslegung oder eine Ölkapselung dieses rückseitigen Gehäuseaufsatzes realisiert werden. Eine weitere, nicht dargestellte Alternative kann die Anordnung des Vorschaltgeräts außerhalb der Anschlusskammer und des Emissionsraums vorsehen, sodass sich lediglich die Anschlussleitungen 22 in der Anschlusskammer 15 befinden und sich zu der oder durch die Anschlussöffnung 16b erstrecken können. D. h., dass elektrische bzw. elektronische Vorrichtungen wie Gleichspannungswandler oder andere Vorschaltgeräte ggf. auch in einem separaten Gehäuse in der Nähe des Flächenstrahler-Gehäusekörpers ausgelagert werden können. Für den Fall, dass der Flächenstrahler 100 in einer ex-klassifizierten Zone betrieben wird, kann dann das separate Gehäuse außerhalb der ex-klassifizierten Zone montiert werden.

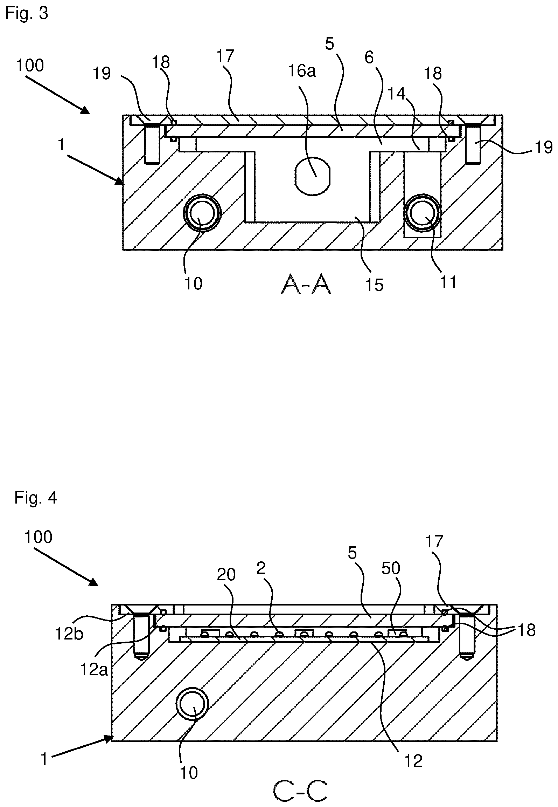

Ein Anschlussstecker 23, der in **Fig. 10** mit dem Vorschaltgerät 24 verbunden ist, alternativ aber auch mit Anschlussleitungen 22 verbunden sein kann, erstreckt sich von der Anschlusskammer 15 durch die Durchtrittöffnung 16a, deren Querschnittfläche kleiner ist als die der Anschlussöffnung 16b, und durch die Anschlussöffnung 16b, sodass von außen der elektrische Anschluss des Flächenstrahlers 100 erfolgen kann. Der Anschlussstecker 23 kann dabei einen Abschnitt aufweisen, dessen Querschnitt mit der Querschnittsfläche der Durchtrittöffnung 16a korrespondiert, die, wie in **Fig. 3** zu sehen, von der Kreisform abweichen kann und im dort dargestellten Beispiel als Zweiflach ausgebildet ist. Anders als in **Fig. 10** dargestellt, kann ein Anschlussstecker 23 eine abgesetzte Form aufweisen, sodass der Anschlussstecker 23 nicht nur einen an den Querschnitt der Durchtrittöffnung 16a angepassten Abschnitt, sondern auch einen an den Querschnitt der Anschlussöffnung 16b angepassten Abschnitt aufweist, um die Anschlusskammer 15 abzudichten. Alternativ oder zusätzlich kann zur Abdichtung der Anordnung des Anschlusssteckers 23 in der Anschlussöffnung 16b und/oder in der Durchtrittöffnung 16a eine Verguss- oder Lotmasse eingesetzt werden, um Leckagen durch die Isolation des Anschlusssteckers 23 zu vermeiden. Entsprechendes gilt für Ausführungsformen, bei denen die Anordnung mit Anschlussleitungen 22 oder mit einem die Anschlussleitungen 22 umfassendes Kabel, die sich durch die Durchtrittöffnung 16a und die Anschlussöffnung 16b erstrecken, abzudichten ist, um Leckagen des Kühlmittels aus der Anschlusskammer 15 zu vermeiden, sodass kein Kühlmittel aufgrund von Kapillareffekten durch die Isolation oder den Schirm der elektrischen Leitung nach außen dringen kann.

In ähnlicher Weise kann auch die Anordnung eines Zulaufanschlusselements 30, das in der im Gehäusekörper 1 ausgebildeten Zulauföffnung 3 angeordnet ist und damit den Zulaufanschluss des Flächenstrahlers 100 bereitstellt, und eines Rücklaufanschlusselements 40, das in der im Gehäusekörper 1 ausgebildeten Rücklauföffnung 4 angeordnet ist und damit den Rücklaufanschluss des Flächenstrahlers 100 bereitstellt (vgl. **Fig. 1****,** **2****,** **5****,** **7, 8****),** gegen Leckagen mittels einer Verguss- oder Lotmasse abgedichtet werden.

Grundsätzlich können alle Anschlussstellen fluiddicht ausgeführt werden, um zu verhindern, dass Kühlmittel durch Kapillareffekte entlang den jeweiligen Anschlusselementen nach außerhalb des Flächenstrahlers 100 dringen kann. Hierzu können Maßnahme, wie beispielsweise das Eingießen von Leitungsabschnitten in den Gehäusekörp1 und/oder mittels Dichtungen abgedichtete Steckverbindungen gegebenenfalls ausreichen. Ferner können bevorzugt sämtliche elektrischen Verbindungen, die von dem Kühlmittel umgeben sind oder damit in Kontakt kommen, fluiddicht ausgeführt sein, um zu vermeiden, dass das Kühlmittel infolge von Kriech- und Kapillareffekten aufgrund der Oberflächenspannung beispielsweise zwischen Kontaktierungsstellen dringt, wo unter Umständen der elektrische Kontakt verschlechtert oder unterbrochen werden könnte. Bei Steckverbindungen können abgedichtete Stecker unter Umständen ausreichen, gegebenenfalls sind aber auch weitere Maßnahmen, z. B. ein Verlöten der Kontaktstellen erforderlich, um nicht nur um ein Kriechen des Kühlmittels zu vermeiden, sondern auch den elektrischen Kontakt sicherzustellen.

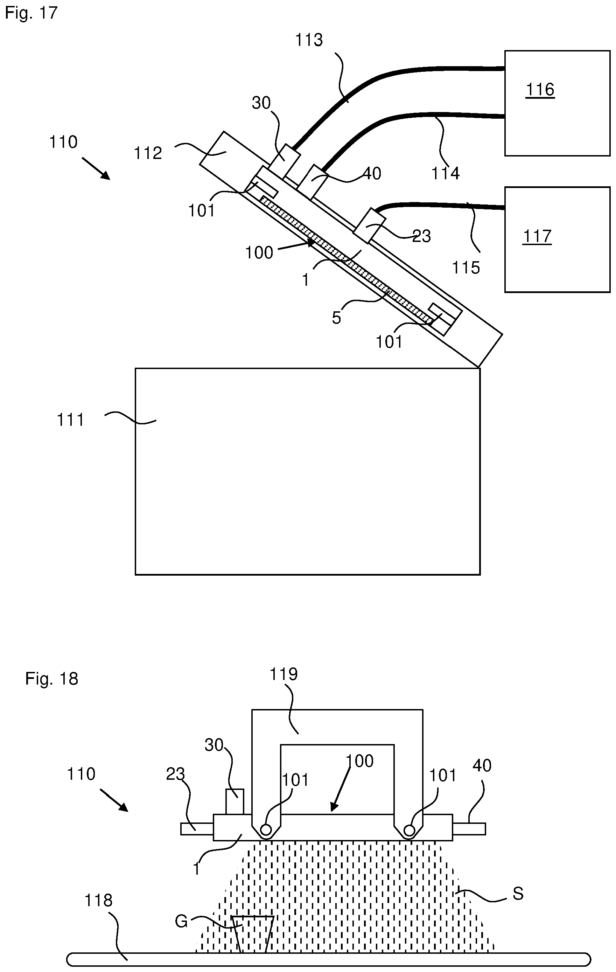

Ein erfindungsgemäßer Flächenstrahler 100 kann - je nach Emissionsspektrum der eingesetzten LEDs 2 - zur Beleuchtung, zur Durchführung einer photochemischen Reaktion oder zur Desinfektion eingesetzt werden, weshalb die Anordnung der Zulauföffnung 3 und der Rücklauföffnung 4 sowie der Anschlussöffnung 16b am Gehäusekörper 1 von einem Einbaukontext des Flächenstrahlers 100 in einer entsprechenden Beleuchtungs-, photochemischen Reaktor- oder Desinfektionsvorrichtung abhängig sein. So können die Zulauföffnung 3 und die Rücklauföffnung 4 wie im Beispiel von **Fig. 1 bis 10** **und** **12 bis 14** gemeinsam an einer Seitenfläche des Gehäusekörpers 1 angeordnet sein. Auch die Anschlussöffnung 16b kann, wie im Beispiel von **Fig. 1 bis 8** **und** **10** gezeigt, auf derselben Seitenfläche wie die Zulauföffnung 3 und die Rücklauföffnung 4 angeordnet sein. Die beispielhafte Ausführungsform des Flächenstrahlers 100 in **Fig. 11** zeigt eine Anordnung der Zulauföffnung 3 und der Rücklauföffnung 4 an der Rückseite des Gehäusekörpers 1, ebenso wie bei dem Flächenstrahler 100 der Vorrichtung 110 in **Fig. 17****,** wie dort aus den dargestellten Zulauf- und Rücklaufanschlusselement 30, 40 ersichtlich wird, die jeweils mit einer dort nicht dargestellten Zulauf- und Rücklauföffnung verbunden sind. **Fig. 17** zeigt ferner einen Anschlussstecker 23, der in der dort nicht dargestellten Anschlussöffnung auf der Rückseite des Gehäusekörpers 1 angeordnet ist. Zulauf-, Rücklauf- und Anschlussöffnung 3, 4, 16b können allerdings auch an verschiedenen Seiten des Gehäusekörpers 1 angeordnet sein, wie beispielhaft an dem Flächenstrahler 100 der in **Fig. 18** dargestellten Vorrichtung 110 zu sehen ist, wo ein Zulaufanschlusselement 30, das mit einer dort nicht dargestellten Zulauföffnung verbunden ist, auf der Rückseite des Gehäusekörpers 1 angeordnet ist, während das Rücklaufanschlusselement 40, das mit einer dort nicht dargestellten Rücklauföffnung verbunden ist, und der Anschlussstecker 23, der in der dort nicht dargestellten Anschlussöffnung angeordnet ist, an voneinander abgewandten Seitenflächen des Gehäusekörpers 1 angeordnet sind. Eine erfindungsgemäße Vorrichtung 110 ist allerdings nicht auf die in den Beispielen dargestellte Anordnung von Zulauf- Rücklauf- und Anschlussöffnung 3, 4, 16b beschränkt - diese kann sich nach dem jeweiligen Einbaukontext richten und entsprechend variieren.

Dadurch, dass die aktive Kühlung durch den direkten Kontakt der LEDs mit dem Kühlmittel den Einsatz von Flächenstrahlern mit einer Vielzahl an LEDs bzw. deren Betrieb mit höchster Leistung erlaubt, wird eine hohe Leistungsdichte erreicht, die mit Nieder- und Mitteldruckstrahlern im Bereich der Photochemie konkurrieren kann. Das Handhaben und Beherrschen des Wärmehaushalts - kurz thermisches Management - eines LED-Flächenstrahlers, der auch die Prozesstemperatur eines Reaktionsmediums, das an das Emissionsfenster des Flächenstrahlers angrenzt, berücksichtigen muss, ist für eine adäquate Lebensdauer der LED von entscheidender Bedeutung.

**Fig. 17** verdeutlicht ein erstes Beispiel einer erfindungsgemäßen Vorrichtung 110, die zur Durchführung einer photochemischen Reaktion mit einem erfindungsgemäßen Flächenstrahler 100 ausgebildet ist, dessen LEDs 2 ein Emissionsspektrum zur Durchführung der photochemischen Reaktion bereitstellen. Die Vorrichtung 110 zur Durchführung einer photochemischen Reaktion ist ein Photoreaktor, dessen Gehäuse ein Reaktorgefäß 111 und einen Reaktordeckel 112 umfasst. Im dargestellten Beispiel ist der für den Flächenstrahler 100 vorgesehene Einbauplatz im Reaktordeckel 112 vorgesehen - andere Reaktorvorrichtungen können Einbauplätze auch in einer Wand des Reaktorgefäßes 111 vorsehen. Ferner kann eine Reaktorvorrichtung 110 mehrere Einbauplätze für mehrere Flächenstrahler aufweisen. Zum Einbau in den Reaktordeckel 112 weist der Gehäusekörper 1 des Flächenstrahlers 100 Befestigungsbohrungen 101 (vgl. auch **Fig. 1** **und** **10****)** als Einbauelemente auf, sodass der Flächenstrahler 100 mittels Schrauben oder Bolzen in dem Reaktordeckel 112 befestigt werden kann. Um auch hier eine optimale Kühlung der LEDs 2 zu erreichen, indem Wärme außerhalb des Flächenstrahlers 100 abgeführt wird, wird das Kühlmittel, das nicht nur die von den LEDs 2 erzeugte Wärme, sondern auch die Wärme aus exothermen Reaktionen aus dem an das Emissionsfenster 5 angrenzenden Reaktionsraum aufnimmt, im Kreislauf geführt, der von dem Rücklaufanschlusselement 40 über eine Kühlmittelrückleitung 114 in der Kreislaufleitung 116, die eine Pumpe und ggf. Wärmetauscher und/oder Armaturen wie Ventile etc. etwa zur Anpassung des Drucks im Emissionsraum umfasst, und über eine Kühlmittelzuleitung 113 zu dem Zulaufanschlusselement 30 führt. Ferner ist der elektrische Anschluss des Flächenstrahlers 100 mit dem Anschlussstecker 23 über ein Anschlusskabel 115 an eine Stromversorgungs- und Steuerungsvorrichtung 117 dargestellt. Die Stromversorgungs- und Steuerungsvorrichtung 117, die beispielsweise Vorschalt- bzw. Leistungselektronik, Treiber - sofern nicht auf der Platine oder in der Anschlusskammer untergebracht - und Netzteile umfassen kann, kann eine externe Stromversorgungs- und Steuerungsvorrichtung sein.

Bei der **in** **Fig. 18** dargestellten Vorrichtung 110 ist der Flächenstrahler 100 mit den Zu- und Rücklaufanschlusselementen 30, 40 sowie dem Anschlussstecker 23 dargestellt, wobei auch hier die Vorrichtung 110 eine hier nicht dargestellte Kreislaufführung für das Kühlmittel von dem Rücklaufanschlusselement 40 über eine Rück-, Kreislauf-, Zuleitung zu dem Zulaufanschlusselement 30 entsprechend **Fig. 17** aufweisen kann. Ferner kann die Vorrichtung 110 eine dargestellte Stromversorgung für den Flächenstrahler 100 aufweisen, die mit dem Anschlussstecker 23 z. B. über ein Anschlusskabel verbunden werden kann. Die in **Fig. 18** dargestellte beispielhafte Vorrichtung 110 ist zur Desinfektion der Oberfläche von Gegenständen G ausgebildet, wobei die Vorderseite des Flächenstrahlers 100 in Richtung eines Transportbandes 118 zeigt, auf dem die zu desinfizierenden Gegenstände G unter dem Flächenstrahler 100 durchgefördert werden, sodass die im Abstrahlbereich des Flächenstrahlers 100 emittierte Einsatzstrahlung S, die zur Desinfektion im UV-C-Spektralbereich liegt, während des Durchförderns auf die Oberflächen des Gegenstands G trifft und dort vorhandene Keime eliminiert. Der Einbaukontext des Flächenstrahlers 100 in dieser Vorrichtung 110 zur Desinfektion umfasst als Gehäuse eine Halterung 119, mit der der Flächenstrahler 100 im vorbestimmten Abstand über dem Transportband 118 angeordnet ist, dazu weist der Flächenstrahler 100 am Gehäusekörper 1 Befestigungsbohrungen 101 (vgl. auch **Fig. 1** **und** **10****)** auf, in die beispielsweise Stifte oder Schrauben zur Befestigung an der Halterung 119 eingreifen können.

Art und Anzahl der Einbauelemente sollen nicht auf die dargestellten Befestigungsbohrungen beschränkt sein, da auch die Art und Anzahl der Einbauelemente vom Einbaukontext sowie der Größe und Gestaltung des Flächenstrahlers 100 abhängig sein können. Sämtliche Einbauelemente können zur Ausbildung einer - vorzugsweise lösbaren - Steck-, Schraub-, Steckschraub-, Klemmverbindung oder ähnlichem ausgeführt sein. Alternative oder zusätzliche nicht dargestellte Einbauelemente können beispielsweise und nicht ausschließlich Stifte, Laschen, Kragen, Stege oder Flansche oder auch Formvorsprünge oder Formausnehmungen zum Eingriff mit entsprechenden Gegenformelementen sein, die den Einbau des Flächenstrahlers 100 in eine Vorrichtung 110 unterstützen oder vereinfachen können.

Der Schutzumfang der vorliegenden Erfindung soll nicht auf die beiden dargestellten Beispiele einer Vorrichtung, die einen erfindungsgemäßen Flächenstrahler 100 umfassen, beschränkt sein.

Ein erfindungsgemäßer Flächenstrahler mit verbesserter Kühlung kann jeglicher Verwendung zur Beleuchtung, zur Durchführung einer photochemischen Reaktion oder zur Desinfektion mit LED, deren Emissionsspektrum eine entsprechende Einsatzstrahlung zur Beleuchtung, zur Durchführung einer photochemischen Reaktion oder zur Desinfektion dienen. Entsprechend sind auch Vorrichtungen zur Beleuchtung sowie von den dargestellten Beispielen abweichende Vorrichtung zur Durchführung einer photochemischen Reaktion oder zur Desinfektion, die einen erfindungsgemäßen Flächenstrahler umfassen, vom Schutzumfang umfasst.

In Bezug auf den Betrieb eines erfindungsgemäßen Flächenstrahlers 100 mit Kreislaufführung des Kühlmittels in einem Photoreaktor bzw. einer Vorrichtung zur Durchführung einer photochemischen Reaktion ist darauf zu achten, dass die Fließgeschwindigkeit, insbesondere bei Silikonöl, gering, d. h. bevorzugt unter 1 m/s gehalten wird, da es ansonsten bei einem im Photoreaktor vorliegenden Produkt, das nicht leitfähig ist, zu einer elektrostatischen Aufladung mit der Gefahr der Bildung einer Zündquelle kommen kann. Um den aktuellen Anforderungen für eine ATEX-Zulassung bezüglich der Zündschutzart "o" = Ölkapselung zu genügen, muss das Kühlmittel eine kinematische Viskosität (bei 25 °C) von zumindest 20 cSt aufweisen, auch wenn Kühlmittel mit geringeren Viskosität von beispielsweise 5 cSt technisch in Bezug auf die Kreislaufführung und Einhaltung der Oberflächentemperaturen der LED vorteilhafter wären. Daher kann das Kühlmittel eine kinematische Viskosität (bei 25 °C) von 5 bis 60 cSt aufweisen, wobei in Bezug auf die geltenden Normen hinsichtlich des Explosionsschutzes eine Viskosität im Bereich von 20 bis 50 cSt bevorzugt ist, um eine entsprechende Zertifizierung zu erhalten. Bei Einsatz von Kühlmittel mit hoher Viskosität ist es ferner vorteilhaft, die Rücklauf- und Zulaufanschlussleitungen zur Vermeidung interner Druckverluste mit ausreichend großem Durchmesser zu dimensionieren, um einen zu hohen Druck an den LEDs zu vermeiden.

Um die Oberflächengrenztemperatur der LEDs einzuhalten, kann der Flächenstrahler bzw. die übergeordnete Vorrichtung in einer weiteren Ausführungsform zur Kontrolle der Fließgeschwindigkeit des Kühlmittels, da das Fließvolumen des im Kreislauf geführten Kühlmittels einen vorbestimmten Mindestwert nicht unterschreiten sollte, zusätzlich einen Durchflussmesser aufweisen, der mit einer Steuerungseinheit verbunden ist, die dazu konfiguriert ist, eine mit einer der Rücklauf- und Zulaufanschlussleitungen verbundenen Pumpe und/oder Armatur (Ventil) in Abhängigkeit des von dem Durchflussmesser gemessenen Durchflusswerts zu steuern, um eine vorbestimmte Fließgeschwindigkeit des Kühlmittels durch den Emissionsraum entlang einer Oberfläche der LED einzuhalten. Die Steuerungseinheit kann eine separate Einheit, Teil des Durchflussmessers, der Pumpe oder der Armatur oder Teil einer Steuerungsvorrichtung der übergeordneten Vorrichtung sein. Bevorzugt können aufgrund der für den Explosionsschutz erforderlichen hohen Viskosität und der gering zu haltenden Fließgeschwindigkeit Massendurchflussmesser, beispielsweise ein Coriolis-Massendurchflussmesser oder Schwebekörperdurchflussmesser oder andere geeignete Messverfahren eingesetzt werden, wohingegen Vortexmessgeräte eher ungeeignet sind. Für die zur ATEX-Zertifizierung erforderlichen hohen Viskositäten und geringen Fließgeschwindigkeiten sind als Durchflussmesser insbesondere Coriolis-Massendurchflussmesser geeignet, die die Sicherheitsstufe ("safety integrity level", kurz SIL) für eine ATEX-Zulassung erfüllen. Aber auch die alternativ einsetzbaren Schwebekörperdurchflussmesser liefern bei hoher Viskosität und geringer Fließgeschwindigkeit des Kühlmittels zuverlässige Messwerte. Ferner kann in einer der im Kreislauf- bzw. Kühlmittelanschlussleitungen, vorzugsweise an einer höchstgelegenen Stelle, eine Atmungseinheit mit Trocknungsmitteln vorgesehen sein, um zur Vermeidung von Kondenswasser eine Entlüftung des Kühlmittels zu ermöglichen, wobei ein Eintrag von Feuchtigkeit durch Frischluft durch ein Trocknungsmittel wie beispielsweise Silicagel vermieden werden kann.

Um die LEDs von den Bedingungen, die in der an das Emissionsfenster angrenzenden Umgebung herrschen, abzukoppeln und die Funktionsfähigkeit der LEDs nicht zu beeinträchtigen, kann das Emissionsfenster ein doppelwandiges Emissionsfenster sein, oder der Flächenstrahler bzw. die übergeordnete Vorrichtung kann ein zweites Emissionsfenster aufweisen, das das den Emissionsraum begrenzende Emissionsfenster überdeckt. Der zwischen den beiden Emissionsfenstern oder den Doppelwänden gebildete Spalt stellt eine weitere thermische Entkopplung bereit. Diese kann noch verstärkt werden, indem in dem Spalt mit einer Absaugvorrichtung ein Unterdruck erzeugt wird oder indem ein weiterer Kühlkreis zur Fluidkühlung in dem Spalt verbunden wird. Bei der Ausführung als doppelwandiges Emissionsfenster kann der Spalt zwischen den Wänden auch schon bei dessen Herstellung evakuiert werden. Als Kühlfluid eignen sich alle für die Einsatzstrahlung transparenten flüssigen oder gasförmigen Fluide, beispielsweise Wasser, Inertgas wie Stickstoff oder auch Luft.

In einigen Ausführungsformen kann ein erfindungsgemäßer Flächenstrahler - abhängig auch von der Anzahl der eingesetzten LEDs - mehrere Platinen mit jeweils einer Teilanzahl der Gesamtheit der LED aufweisen, wobei die Platinen einzeln ansteuerbar sind. Dies ermöglicht, dass bei Ausfall einer LED auf einer Platine nicht alle LEDs abgeschaltet werden müssen, sondern lediglich die betroffene Platine, während die anderen Platinen weiter in Betrieb bleiben können. Ein Austausch der betroffenen Platine kann auf einen geeigneten Zeitpunkt verschoben werden, etwa nachdem eine mit dem Flächenstrahler durchgeführte photochemische Reaktion beendet wurde. Zu diesem Zweck kann der Flächenstrahler pro Platine eine Erkennungseinheit aufweisen, die dazu konfiguriert ist, einen Ausfall einer oder mehrerer LED(s) auf einer Platine festzustellen und in Abhängigkeit eines festgestellten Ausfalls die Stromversorgung für die betroffene Platine zu unterbrechen und ggf. für die weiteren Platinen korrespondierend zu begrenzen. Ggf. kann über die Steuerungsvorrichtung des Flächenstrahlers bzw. der übergeordneten Vorrichtung auch eine Warnmeldung ausgegeben werden, wenn die Erkennungseinheit mit der Stromversorgungs- und Steuerungsvorrichtung verbunden ist. Grundsätzlich ist eine solche Erkennungseinheit auch pro LED denkbar, sodass bei Ausfall einzelner LEDs die jeweilige Stromversorgung unterbrochen und die Stromversorgung für die weiteren LEDs ggf. korrespondierend begrenzt wird, sodass vermieden wird, dass ein LED-Ausfall, der von einem Temperatur-Hotspot begleitet wird, zu einer Kettenreaktion mit dem Ausfall weiterer LEDs infolge überhöhter Grenztemperatur führt.

Ferner kann der Flächenstrahler einen oder mehrere Temperatursensor(en) aufweisen, der/die auf dem Gehäusekörper oder einer Platine angeordnet und mit der Stromversorgungs- und Steuerungsvorrichtung verbunden ist/sind, die einen Schutzschalter für LEDs umfasst. Der Schutzschalter sorgt für eine Schutzabschaltung zum Schutz der Halbleiterbauteile, wenn die maximal zulässige Umgebungstemperatur überschritten wird. Sind die LEDs bzw. LED-Gruppen eines Flächenstrahlers einzeln ansteuerbar, und ist jeweils ein Temperatursensor einer LED bzw. LED-Gruppe zugeordnet, kann die Steuerungsvorrichtung, wenn einer der Sensoren ein Überschreiten der Maximaltemperatur feststellt, die diesem Sensor zugeordnete LEDs bzw. die LED-Gruppe abschalten. Entsprechend kann die Steuerungsvorrichtung die entsprechende LED/LED-Gruppe wieder selbsttätig zuschalten, wenn durch den Sensor festgestellt wird, dass als Folge der Schutzabschaltung die Maximaltemperatur unterschritten wird. Sämtliche sicherheitsrelevanten Sensoren des Flächenstrahlers wie die Erkennungseinheiten und Temperatursensoren, können redundant bzw. zweikanalig ausgeführt sein, um die entsprechend notwendige SIL Klasse zu realisieren.

Die Stromversorgungs- und Steuerungsvorrichtung kann ferner alternativ oder zusätzlich zumindest einen Regelkreis für die LED-Ansteuerung aufweisen, mit dem gleichartige oder unterschiedliche LEDs dimmbar sind und/oder das Spektrum der emittierten Wellenlängen unterschiedlicher LEDs änderbar ist, um die emittierte Lichtmenge oder die emittierten Wellenlängen nach Wunsch oder Erfordernis anzupassen. Damit können einsatz- bzw. prozessspezifische Spektren bereitgestellt werden, wobei ferner mittels Regelkreis die Strahlungsintensität dem Einsatz, z. B. photochemischen Prozess, angepasst werden kann. So kann z. B. in einer Vorrichtung zur Durchführung einer photochemischen Reaktion eine Leistungsregelung der LED (Dimming) zur Prozesskontrolle erfolgen, da in vielen Reaktionen sich die Absorption während des Prozesses ändert. Hierauf kann durch gezielte Mess- und Regelkreise und LED-Dimming reagiert werden, um ein effizientes System zu realisieren und Überbestrahlungen zu vermeiden.

Ein erfindungsgemäßer Flächenstrahler kann monochromatische LEDs sowie eine Mischung von LEDs mit unterschiedlichen Emissionsspektren aufweisen, die eine optimale Einsatzstrahlung liefern, was bei der Durchführung einer photochemischen Reaktion oder Desinfektion einer optimale Ausnutzung des Absorptionsspektrums der jeweiligen Reaktion entspricht. Ähnliches gilt, wenn die Vorrichtung zur Durchführung einer photochemischen Reaktion ein Bioreaktor ist. Hier können LEDs mit unterschiedlichen Emissionswellenlängen implementiert werden, um optimale Wachstumsraten zu erzielen. In unterschiedlichen Wachstumsphasen bzw. für unterschiedliche Zellen können jeweils optimal gemischte Lichtspektren und -intensitäten zum optimierten Wachstum eingesetzt werden.

### BEZUGSZEICHENLISTE

- 1: Gehäusekörper
- 2: Lichtemittierendes Halbleiterbauelement (LED)
- 2a, 2b, 2c,: Primäroptik, Halbleiterkristall, Draht,
- 2d, 2e, 2f, 2g: Anode, LED-Chip, Kathode, Basisstruktur
- 3: Zulauföffnung
- 4: Rücklauföffnung
- 5: Emissionsfenster
- 6: Emissionsraum
- 10, 10a, 10b: Erster Kühlkanal, zulaufnaher, zulaufferner Verteilerkanalabschnitt
- 11, 11a: Zweiter Kühlkanal, Sammelkanalabschnitt
- 12: Befestigungsfläche
- 12a, 12b: Befestigungsabsatz
- 13: Mündungsöffnung
- 14: Ablauföffnung
- 15: Anschlusskammer
- 16a, 16b: Durchtrittöffnung, Anschlussöffnung
- 17, 17a: Halterungsrahmen, Öffnung
- 18, 18a: Dichtung, Dichtungsnut
- 19: Befestigungsbohrung
- 20: Platine
- 21: Anschlusskontaktpaar
- 22: Anschlussleitung
- 23: Anschlussstecker
- 24: Treiber/Vorschaltgerät
- 30: Zulaufanschlusselement
- 40: Rücklaufanschlusselement
- 50: Befestigungsmittel
- 60: Klebstoffschicht
- 100: Flächenstrahler
- 101: Einbauelement
- 110: Vorrichtung zur Beleuchtung, zur Durchführung einer photochemischen Reaktion oder zur Desinfektion
- 111: Reaktorgefäß
- 112: Reaktordeckel
- 113: Kühlmittelzuleitung
- 114: Kühlmittelrückleitung
- 115: Anschlusskabel
- 116: Kreislaufleitung (mit Pumpe und ggf. Wärmetauscher)
- 117: Stromversorgungs- und Steuerungsvorrichtung
- 118: Förderband
- 119: Halterung
- a: Fluidweg
- G: Behandlungsgegenstand
- K: elektrisch isolierendes Kühlmittel
- S: Einsatzstrahlung

## Patentansprüche

1. Flächenstrahler (100), der zumindest ein lichtemittierendes Halbleiterbauelement (2) und einen Gehäusekörper (1) aufweist, wobei
- der Gehäusekörper (1) zumindest einen Kühlkanal (10, 11) für ein Kühlmittel aufweist, der zumindest einen Teil eines Fluidwegs (a) bildet, der sich von einer Zulauföffnung (3) zu einer Rücklauföffnung (4) erstreckt, die an dem Gehäusekörper (1) ausgebildet sind, und
- an dem Gehäusekörper (1) ein Emissionsfenster (5) angeordnet ist, das zumindest ein lichtemittierendes Halbleiterbauelement (2) überlagert, und das eine Vorderseite des Flächenstrahlers (100) definiert und das für eine von dem Halbleiterbauelement (2) emittierbare Einsatzstrahlung (S) transparent ist, und
- der Gehäusekörper (1) eine von dem Emissionsfenster (5) beabstandete Befestigungsfläche (12) für das lichtemittierende Halbleiterbauelement (2) bereitstellt, wobei die Anordnung des Emissionsfensters (5) an dem Gehäusekörper (1) fluiddicht ausgebildet ist und der Gehäusekörper (1), das zumindest eine lichtemittierende Halbleiterbauelement (2) und das Emissionsfenster (5) einen Emissionsraum (6) begrenzen,
wobei der Fluidweg (a) definiert wird durch
i) zumindest einen ersten Kühlkanal (10), der sich von der Zulauföffnung (3) durch den Gehäusekörper (1) zu zumindest einer Mündungsöffnung (13) erstreckt, die auf einer ersten Seite benachbart zu der Befestigungsfläche (12) ausgebildet ist, und
ii) den Emissionsraum (6) von der zumindest einen Mündungsöffnung (13) bis zu zumindest einer Ablauföffnung (14), die auf einer von der ersten Seite abgewandten zweiten Seite benachbart zu der Befestigungsfläche (12) ausgebildet ist, und
iii) zumindest einen zweiten Kühlkanal (11), der sich von der Ablauföffnung (14) durch den Gehäusekörper (1) zu der Rücklauföffnung (4) erstreckt,
wobei das Kühlmittel (K) eine elektrisch isolierende Flüssigkeit ist, die für die Einsatzstrahlung (S) transparent ist,
**dadurch gekennzeichnet, dass**
an der Vorderseite in dem Gehäusekörper (1) benachbart zu der Befestigungsfläche (12) eine zu dem Emissionsraum (5) geöffnete Anschlusskammer (15) ausgebildet ist, wobei an dem Gehäusekörper (1) eine Anschlussöffnung (16b) ausgebildet ist, die mit der Anschlusskammer (15) verbunden ist, wobei sich zumindest eine Anschlussleitung (22) zum Anschluss des zumindest einen lichtemittierenden Halbleiterbauelements (2) zumindest in die Anschlusskammer (15) erstreckt,
wobei
- ein Vorschaltgerät auf einer Platine (20) vorgesehen ist, mittels derer eine Mehrzahl der lichtemittierenden Halbleiterbauelemente (2) an der Befestigungsfläche (12) befestigt ist und auf der jeweils ein Paar Anschlusskontakte (21) für eine Reihe lichtemittierender Halbleiterbauelemente (2) an einer der Anschlusskammer (15) nahen Seite der Platine (20) ausgebildet ist, wobei jeder Anschlusskontakt (21) mit einer jeweiligen Anschlussleitung (22) verbunden ist und das Vorschaltgerät zwischen den Anschlusskontakten (21) und den lichtemittierenden Halbleiterbauelementen (2) vorgesehen ist, oder
- ein Vorschaltgerät (24) für das zumindest eine lichtemittierende Halbleiterbauelement (2) verbunden mit der zumindest einen Anschlussleitung (22) in der Anschlusskammer (15) angeordnet ist.

2. Flächenstrahler (100) nach Anspruch 1,
**dadurch gekennzeichnet, dass**
der Gehäusekörper (1), der zur Anordnung des Flächenstrahlers (100) in einer Vorrichtung (110) zur Beleuchtung, zur Durchführung einer photochemischen Reaktion oder zur Desinfektion ausgebildet ist, eine von der Vorderseite abgewandte Rückseite aufweist und zwischen Vorderseite und Rückseite durch Seitenflächen begrenzt wird, wobei die Zulauföffnung (3) und die Rücklauföffnung (4) gemeinsam an einer der Seitenflächen oder der Rückseite, oder einzeln an verschiedenen Seitenflächen oder einer der Seitenfläche und der Rückseite angeordnet sind.

3. Flächenstrahler (100) nach Anspruch 1 oder 2,
**dadurch gekennzeichnet, dass**
die zumindest eine Mündungsöffnung (13) an einer von der Zulauföffnung (3) entfernten Seite der Befestigungsfläche (12) ausgebildet ist, wobei der erste Kühlkanal (10) zumindest teilweise in einer Ebene parallel zu der Befestigungsfläche (12) durch den Gehäusekörper (1) verläuft,
wobei bevorzugt die zumindest eine Ablauföffnung (14) an einer der Rücklauföffnung (4) nahen Seite der Befestigungsfläche (12) ausgebildet ist

4. Flächenstrahler (100) nach zumindest einem der Ansprüche 1 bis 3,
**dadurch gekennzeichnet, dass**
- die Zulauföffnung (3) mit einem der Zulauföffnung (3) nahen Verteilerkanalabschnitt (10a) fluidisch verbunden ist, von dem sich mehrere erste Kühlkanäle (10) zu entsprechenden mehreren Mündungsöffnungen (13) auf einer von der Zulauföffnung (3) entfernten Seite der Befestigungsfläche (12) erstrecken, und der zweite Kühlkanal (11) sich von einen Sammelkanalabschnitt (11a), der mit einer Mehrzahl Ablauföffnungen (14) verbunden ist, zu der Rücklauföffnung (4) erstreckt,
oder
- der erste Kühlkanal (10) sich von der Zulauföffnung (3) zu einem von der Zulauföffnung (3) entfernten Verteilerkanalabschnitt (10b) erstreckt, an dem eine Mehrzahl Mündungsöffnungen (13) ausgebildet ist, und der zweite Kühlkanal (11) sich von einen Sammelkanalabschnitt (11a), der mit einer Mehrzahl Ablauföffnungen (14) verbunden ist, zu der Rücklauföffnung (4) erstreckt.

5. Flächenstrahler (100) nach zumindest einem der Ansprüche 1 bis 4,
**dadurch gekennzeichnet, dass**
- der Flächenstrahler (100) einen Halterungsrahmen (17) aufweist, der an dem Gehäusekörper (1) zur Halterung des Emissionsfensters (5) angeordnet und dazu ausgebildet ist, das zumindest ein lichtemittierendes Halbleiterbauelement (2) unbedeckt zu lassen, wobei jeweils eine umlaufende Dichtung (18) zwischen dem Halterungsrahmen (17) und dem Emissionsfenster (5) und zwischen dem Emissionsfenster (5) und dem Gehäusekörper (1) angeordnet ist,
und/oder
- das Emissionsfenster (5) durch eine Klebstoffschicht (60) an dem Gehäusekörper (1) befestigt ist

6. Flächenstrahler (100) nach zumindest einem der Ansprüche 1 bis 5,
**dadurch gekennzeichnet, dass**
der Gehäusekörper (1) an der Vorderseite zumindest einem Befestigungsabsatz (12a, 12b) aufweist, der die Befestigungsfläche (12) umgibt, wobei ein erster Befestigungsabsatz (12a) zur Aufnahme des Emissionsfensters (5) ausgebildet ist und der Abstand des ersten Befestigungsabsatzes (12a) von der Befestigungsfläche (12) eine Höhe des Emissionsraums (6) definiert.

7. Flächenstrahler (100) nach zumindest einem der Ansprüche 1 bis 6,
**dadurch gekennzeichnet, dass**
die Anschlussöffnung (16b) über eine Durchtrittöffnung (16a) mit der Anschlusskammer (15) verbunden ist, wobei eine Querschnittfläche der Durchtrittöffnung (16a) kleiner als eine Querschnittfläche der Anschlussöffnung (16b) ist.

8. Flächenstrahler (100) nach zumindest einem der Ansprüche 1 bis 7,
**dadurch gekennzeichnet, dass**
die zumindest eine Anschlussleitung (22) sich zumindest teilweise in die Anschlussöffnung (16b) erstreckt, wobei die Anordnung der zumindest einen Anschlussleitung (22) in der Anschlussöffnung (16b) durch eine Verguss- oder Lotmasse abgedichtet ist,
oder
ein Anschlussstecker (23) mit der zumindest einen Anschlussleitung (22) verbunden ist, wobei der Anschlussstecker (23) sich zumindest teilweise in die Anschlussöffnung (16b) erstreckt, wobei die Anordnung des Anschlusssteckers (23) in der Anschlussöffnung (16b) durch eine Verguss- oder Lotmasse abgedichtet ist.

9. Flächenstrahler (100) nach zumindest einem der Ansprüche 1 bis 8,
**dadurch gekennzeichnet, dass**
der Flächenstrahler (100) ein Zulaufanschlusselement (30), das mit der Zulauföffnung (3) verbunden ist, und ein Rücklaufanschlusselement (40) aufweist, das mit der Rücklauföffnung (4) verbunden ist, wobei die Verbindung des Zulaufanschlusselements (30) mit der Zulauföffnung (3) und/oder des Rücklaufanschlusselements (40) mit der Rücklauföffnung (4) durch eine Verguss- oder Lotmasse abgedichtet ist.

10. Vorrichtung (110) zur Beleuchtung, zur Durchführung einer photochemischen Reaktion oder zur Desinfektion, die einen Flächenstrahler (100) mit zumindest einem lichtemittierenden Halbleiterbauelement (2) aufweist, dessen Emissionsspektrum eine entsprechende Einsatzstrahlung (S) zur Beleuchtung, zur Erwärmung, zur Durchführung einer photochemischen Reaktion oder zur Desinfektion bereitstellt, **dadurch gekennzeichnet, dass**
der Flächenstrahler ein Flächenstrahler (100) nach zumindest einem der Ansprüche 1 bis 9 ist.

11. Vorrichtung (110) nach Anspruch 10,
**dadurch gekennzeichnet, dass**
die Vorrichtung (110) ein Gehäuse (111, 112, 119) aufweist, das einen Beleuchtungsraum, Reaktionsraum oder Desinfektionsraum zumindest teilweise umgibt und das zumindest einen Einbauplatz für den zumindest einen Flächenstrahler (100) aufweist, dessen Gehäusekörper (1) vorzugsweise zumindest ein Einbauelement (101) zur Anordnung in der Vorrichtung (110) aufweist.

12. Verwendung des Flächenstrahlers (100) nach zumindest einem der Ansprüche 1 bis 9 zur Beleuchtung, zur Durchführung einer photochemischen Reaktion oder zur Desinfektion, wobei das zumindest eine lichtemittierende Halbleiterbauelement (2) ein Emissionsspektrum aufweist, das eine entsprechende Einsatzstrahlung (S) zur Beleuchtung, zur Durchführung einer photochemischen Reaktion oder zur Desinfektion bereitstellt.

## Claims

1. A surface radiator (100), which has at least one light-emitting semiconductor component (2) and a housing body (1), wherein
- the housing body (1) has at least one cooling channel (10, 11) for a coolant, which forms at least a part of a fluid path (a), which extends from an inlet opening (3) to a return opening (4), which are formed on the housing body (1), and
- an emission window (5), which overlies at least one light-emitting semiconductor component (2) and which defines a front side of the surface radiator (100) and which is transparent for an incident radiation (S), which can be emitted by the semiconductor component (2), is arranged on the housing body (1), and
- the housing body (1) provides a fastening surface(12), which is spaced apart from the emission window (5), for the light-emitting semiconductor component (2), wherein the arrangement of the emission window (5) on the housing body (1) is formed in a fluid-tight manner and the housing body (1), the at least one light-emitting semiconductor component (2) and the emission window (5) delimit an emission chamber (6),
wherein the fluid path (a) is defined by
i) at least one first cooling channel (10), which extends from the inlet opening (3) through the housing body (1) to at least one orifice opening (13), which is formed on a first side adjacent to the fastening surface (12) and
ii) the emission chamber (6) from the at least one orifice opening (13) all the way to at least one discharge opening (14), which is formed on a second side facing away from the first side adjacent to the fastening surface (12) and
iii) at least one second cooling channel (11), which extends from the discharge opening (14) through the housing body (1) to the return opening (4),
wherein the coolant (K) is an electrically insulating liquid, which is transparent for the incident radiation (S),
**characterized in that**
a connecting chamber (15), which is open to the emission chamber (5), is formed on the front side in the housing body (1) adjacent to the fastening surface (12), wherein a connecting opening (16b), which is connected to the connecting chamber (15), is formed on the housing body (1), wherein at least one connecting line (22) for connecting the at least one light-emitting semiconductor component (2) extends at least into the connecting chamber (15),
wherein
- a ballast is provided on a printed circuit board (20), by means of which a plurality of the light-emitting semiconductor components (2) is fastened to the fastening surface (12) and on which a pair of connecting contacts (21) for a row of light-emitting semiconductor components (2) is in each case formed on a side of the printed circuit board (20) close to the connecting chamber (15), wherein each connecting contact (21) is connected to a respective connecting line (22) and the ballast is provided between the connecting contacts (21) and the light-emitting semiconductor components (2) or
- a ballast (24) for the at least one light-emitting semiconductor component (2) is arranged in the connecting chamber (15), connected to the at least one connecting line (22).

2. The surface radiator (100) according to claim 1,
**characterized in that**
the housing body (1), which is formed for the arrangement of the surface radiator (100) in a device (110) for lighting, for carrying out a photochemical reaction or for disinfection, has a rear side facing away from the front side and is delimited between front side and rear side by means of side surfaces, wherein the inlet opening (3) and the return opening (4) are arranged jointly on one of the side surfaces or the rear side or individually on different side surfaces or on one of the side surfaces and the rear side.

3. The surface radiator (100) according to claim 1 or 2,
**characterized in that**
the at least one orifice opening (13) is formed on a side of the fastening surface (12) facing away from the inlet opening (3), wherein the first cooling channel (10) runs through the housing body (1) at least partly in a plane parallel to the fastening surface (12),
wherein the at least one discharge opening (14) is preferably formed on a side of the fastening surface (12) close to the return opening (4).

4. The surface radiator (100) according to at least any one of claims 1 to 3, **characterized in that**
- the inlet opening (3) is fluidically connected to a distributor channel section (10a) close to the inlet opening (3), from which several first cooling channels (10) extend to corresponding orifice openings (13) on a side of the fastening surface (12) spaced apart from the inlet opening (3), and the second cooling channel (11) extends from a collecting channel section (11a), which is connected to a plurality of discharge openings (14), to the return opening (4),
or
- the first cooling channel (10) extends from the inlet opening (3) to a distributor channel section (10b) spaced apart from the inlet opening (3), on which a plurality of orifice openings (13) is formed, and the second cooling channel (11) extends from a collecting channel section (11a), which is connected to a plurality of discharge openings (14), to the return opening (4).

5. The surface radiator (100) according to at least any one of claims 1 to 4,
**characterized in that**
- the surface radiator (100) has a holding frame (17), which is arranged on the housing body (1) for holding the emission window (5) and which is formed for leaving the at least one light-emitting semiconductor component (2) uncovered, wherein a respective circumferential seal (18) is arranged between the holding frame (17) and the emission window (5) and between the emission window (5) and the housing body (10),
and/or
the emission window (5) is fastened to the housing body (1) by means of an adhesive layer (60).

6. The surface radiator (100) according to at least any one of claims 1 to 5,
**characterized in that**
on the front side, the housing body (1) has at least one fastening ledge (12a, 12b), which surrounds the fastening surface (12), wherein a first fastening ledge (12a) is formed for receiving the emission window (5) and the distance of the first fastening ledge (12a) from the fastening surface (12) defines a height of the emission chamber (6).

7. The surface radiator (100) according to at least any one of claims 1 to 6,
**characterized in that**
the connecting opening (16b) is connected to the connecting chamber (15) via a passage opening (16a), wherein a cross sectional surface of the passage opening (16a) is smaller than a cross sectional surface of the connecting opening (16b).

8. The surface radiator (100) according to at least any one of claims 1 to 7,
**characterized in that**
the at least one connecting line (22) extends at least partly into the connecting opening (16b), wherein the arrangement of the at least one connecting line (22) in the connecting opening (16b) is sealed by means of a casting or solder compound,
or
a connecting plug (23) is connected to the at least one connecting line (22), wherein the connecting plug (23) extends at least partly into the connecting opening (16b), wherein the arrangement of the connecting plug (23) in the connecting opening (16b) is sealed by means of a casting or solder compound.

9. The surface radiator (100) according to at least any one of claims 1 to 8,
**characterized in that**
the surface radiator (100) has an inlet connecting element (30), which is connected to the inlet opening (3), and a return connecting element (40), which is connected to the return opening (4), wherein the connection of the inlet connecting element (30) to the inlet opening (3) and/or of the return connecting element (40) to the return opening (4) is sealed by means of a casting or solder compound.

10. A device (110) for lighting, for carrying out a photochemical reaction or for disinfection, which has a surface radiator (100) comprising at least one light-emitting semiconductor component (2), the emission spectrum of which provides a corresponding incident radiation (S) for lighting, for heating, for carrying out a photochemical reaction or for disinfection,
**characterized in that**
the surface radiator is a surface radiator (100) according to at least any one of claims 1 to 9.

11. The device (110) according to claim 10,
**characterized in that**
the device (110) has a housing (111, 112, 119), which at least partly surrounds a lighting chamber, reaction chamber or disinfection chamber and has at least one installation space for the at least one surface radiator (100), the housing body (1) of which preferably has at least one installation element (101) for the arrangement in the device (110).

12. Use of the surface radiator (100) according to at least any one of claims 1 to 9 for lighting, for carrying out a photochemical reaction or for disinfection, wherein the at least one light-emitting semiconductor component (2) has an emission spectrum, which provides a corresponding incident radiation (S) for lighting, for carrying out a photochemical reaction or for disinfection.

## Revendications

1. Projecteur de surface (100) comprenant au moins un composant semi-conducteur électroluminescent (2) et un corps de boîtier (1),
- le corps de boîtier (1) comportant au moins un canal de refroidissement (10, 11) pour un liquide de refroidissement, lequel forme au moins une partie d'un trajet de fluide (a) s'étendant d'une ouverture d'entrée (3) à une ouverture de retour (4), formées sur le corps de boîtier (1), et
- une fenêtre d'émission (5) étant disposée sur le corps de boîtier (1), laquelle recouvre au moins un composant semi-conducteur électroluminescent (2), définissant une face avant du projecteur de surface (100) et étant transparente au rayonnement d'entrée (S) émis par le composant semi-conducteur (2), et
- le corps de boîtier (1) présentant une surface de fixation (12) pour le composant semi-conducteur électroluminescent (2), espacée de la fenêtre d'émission (5), la disposition de la fenêtre d'émission (5) sur le corps de boîtier (1) étant étanche aux fluides et le corps de boîtier (1), l'au moins un composant semi-conducteur électroluminescent (2) et la fenêtre d'émission (5) délimitant un espace d'émission (6),
le trajet du fluide (a) étant défini par
i) au moins un premier canal de refroidissement (10) s'étendant de l'ouverture d'entrée (3) à travers le corps de boîtier (1) jusqu'à au moins une ouverture d'orifice (13) formée sur un premier côté adjacent à la surface de fixation (12), et
ii) l'espace d'émission (6) depuis au moins une ouverture d'orifice (13) jusqu'à au moins une ouverture de sortie (14) qui est formée sur un deuxième côté opposé au premier côté adjacent à la surface de fixation (12), et
iii) au moins un deuxième canal de refroidissement (11) s'étendant de l'ouverture de sortie (14) à travers le corps de boîtier (1) jusqu'à l'ouverture de retour (4),
le liquide de refroidissement (K) étant un liquide électriquement isolant qui est transparent au rayonnement d'entrée (S),
**caractérisé en ce que**
sur la face avant du corps de boîtier (1), adjacente à la surface de fixation (12), est formée une chambre de jonction (15) ouverte sur l'espace d'émission (5), une ouverture de jonction (16b) qui est reliée à la chambre de jonction (15) étant formée sur le corps de boîtier (1), au moins une ligne de jonction (22) pour connecter l'au moins un composant semi-conducteur électroluminescent (2) s'étendant au moins dans la chambre de jonction (15),
- un ballast étant prévu sur une carte de circuit imprimé (20), au moyen de laquelle plusieurs composants semi-conducteurs électroluminescents (2) sont fixés à la surface de fixation (12) et sur laquelle une paire de contacts de connexion (21) pour une rangée de composants semi-conducteurs électroluminescents (2) est formée sur un côté de la carte de circuit imprimé (20), à proximité de la chambre de jonction (15), chaque contact de connexion (21) étant connecté à une ligne de jonction respective (22) et le ballast étant prévu entre les contacts de connexion (21) et les composants semi-conducteurs électroluminescents (2), ou
- un ballast (24) pour l'au moins un composant semi-conducteur électroluminescent, (2) connecté à l'au moins une ligne de jonction (22), étant disposé dans la chambre de jonction (15).

2. Projecteur de surface (100) selon la revendication 1,
**caractérisé en ce que**
le corps de boîtier (1) qui est conçu pour la disposition du projecteur de surface (100) dans un dispositif (110) pour l'éclairage, pour la mise en œuvre d'une réaction photochimique ou pour la désinfection, présente une face arrière opposée à la face avant et est délimité entre la face avant et la face arrière par des surfaces latérales, l'ouverture d'entrée (3) et l'ouverture de retour (4) étant disposées conjointement sur l'une des surfaces latérales ou sur la face arrière, ou individuellement sur des surfaces latérales différentes ou sur l'une des surfaces latérales et sur la face arrière.

3. Projecteur de surface (100) selon la revendication 1 ou 2,
**caractérisé en ce que**
l'au moins une ouverture d'orifice (13) est formée sur un côté de la surface de fixation (12), éloigné de l'ouverture d'entrée (3), le premier canal de refroidissement (10) s'étendant au moins partiellement dans un plan parallèle à la surface de fixation (12) à travers le corps de boîtier (1)
l'au moins une ouverture de sortie (14) de préférence étant formée sur un côté de la surface de fixation (12), à proximité de l'ouverture de retour (4).

4. Projecteur de surface (100) selon au moins l'une des revendications 1 à 3, **caractérisé en ce que**
- l'ouverture d'entrée (3) est reliée fluidiquement à une section du canal distributeur (10a) à proximité de l'ouverture d'entrée (3), à partir de laquelle plusieurs premiers canaux de refroidissement (10) s'étendent vers plusieurs ouvertures d'orifice (13) correspondantes sur un côté de la surface de fixation (12), éloigné de l'ouverture d'entrée (3), et le deuxième canal de refroidissement (11) s'étend d'une section du canal collecteur (11a), reliée à plusieurs ouvertures de sortie (14), vers l'ouverture de retour (4),
ou
- le premier canal de refroidissement (10) s'étend de l'ouverture d'entrée (3) vers une section du canal distributeur (10b), éloignée de l'ouverture d'entrée (3), sur laquelle plusieurs ouvertures d'orifice (13) sont formées, et le deuxième canal de refroidissement (11) s'étend d'une section du canal collecteur (11a), reliée à plusieurs ouvertures de sortie (14), vers l'ouverture de retour (4).

5. Projecteur de surface (100) selon au moins l'une des revendications 1 à 4, **caractérisé en ce que**
- le projecteur de surface (100) comporte un cadre de maintien (17) disposé sur le corps de boîtier (1) pour maintenir la fenêtre d'émission (5) et conçu pour laisser l'au moins un composant semi-conducteur électroluminescent (2) découvert, un joint circonférentiel (18) étant disposé entre le cadre de maintien (17) et la fenêtre d'émission (5) et entre la fenêtre d'émission (5) et le corps de boîtier (1) et/ou
- la fenêtre d'émission (5) est fixée au corps du boîtier (1) par une couche adhésive (60).

6. Projecteur de surface (100) selon au moins l'une des revendications 1 à 5, **caractérisé en ce que**
le corps de boîtier (1) présente au moins un épaulement de fixation (12a, 12b) sur la face avant, lequel entoure la surface de fixation (12), un premier épaulement de fixation (12a) étant conçu pour recevoir la fenêtre d'émission (5) et la distance du premier épaulement de fixation (12a) par rapport à la surface de fixation (12) définissant une hauteur de l'espace d'émission (6).

7. Projecteur de surface (100) selon au moins l'une des revendications 1 à 6, **caractérisé en ce que**
l'ouverture de jonction (16b) est reliée à la chambre de jonction (15) via une ouverture traversante (16a), une section transversale de l'ouverture traversante (16a) étant inférieure à une section transversale de l'ouverture de jonction (16b).

8. Projecteur de surface (100) selon au moins l'une des revendications 1 à 7, **caractérisé en ce que**
l'au moins une ligne de jonction (22) s'étend au moins partiellement dans l'ouverture de jonction (16b), la disposition de l'au moins une ligne de jonction (22) dans l'ouverture de jonction (16b) étant scellée par un composé de coulée ou de soudure ou
une fiche de connecteur (23) est connectée à l'au moins une ligne de jonction (22), la fiche de connecteur (23) s'étendant au moins partiellement dans l'ouverture de jonction (16b), la disposition de la fiche de connecteur (23) dans l'ouverture de jonction (16b) étant scellée par un composé de coulée ou de soudure.

9. Projecteur de surface (100) selon au moins l'une des revendications 1 à 8, **caractérisé en ce que**
le projecteur de surface (100) comporte un élément de connexion d'entrée (30) relié à l'ouverture d'entrée (3) et un élément de connexion de retour (40) relié à l'ouverture de retour (4), la connexion de l'élément de connexion d'entrée (30) à l'ouverture d'entrée (3) et/ou de l'élément de connexion de retour (40) à l'ouverture de retour (4) étant scellée par un composé de coulée ou de soudure.

10. Dispositif (110) d'éclairage, de mise en œuvre d'une réaction photochimique ou de désinfection, comprenant un projecteur de surface (100) doté d'au moins un composant semi-conducteur électroluminescent (2), dont le spectre d'émission fournit un rayonnement d'entrée (S) correspondant pour l'éclairage, le chauffage, la mise en œuvre d'une réaction photochimique ou la désinfection,
**caractérisé en ce que**
le projecteur de surface est un projecteur de surface (100) selon au moins l'une des revendications 1 à 9.

11. Dispositif (110) selon la revendication 10,
**caractérisé en ce que**
le dispositif (110) comporte un boîtier (111, 112, 119) qui entoure au moins partiellement une chambre d'éclairage, une chambre de réaction ou une chambre de désinfection et qui comporte au moins un emplacement d'installation pour l'au moins un projecteur de surface (100), dont le corps de boîtier (1) comporte de préférence au moins un élément d'installation (101) destiné à être disposé dans le dispositif (110).

12. Utilisation du projecteur de surface (100) selon au moins l'une des revendications 1 à 9 pour l'éclairage, la mise en œuvre d'une réaction photochimique ou la désinfection, l'au moins un composant semi-conducteur électroluminescent (2) présentant un spectre d'émission fournissant un rayonnement d'entrée (S) correspondant pour l'éclairage, la mise en œuvre d'une réaction photochimique ou la désinfection.
